# EUROPEAN PATENT SPECIFICATION

(11) **EP 1 774 029 B1**
(45) Date of publication and mention of the grant of the patent: **24.04.2013**
(21) Application number: 05770038.7
(22) Date of filing: 08.07.2005
(51) Int. Cl.: C12Q 1/68

(54) **METHOD FOR DETECTING THE RISK OF TYPE 2 DIABETES**
VERFAHREN ZUM NACHWEIS DES RISIKOS VON DIABETES TYP 2
PROCEDE POUR DETECTER LES RISQUES DE DIABÈTE DE TYPE 2

(30) Priority: 16.07.2004 US 588345 P
(43) Date of publication of application: 18.04.2007
(73) Proprietor: DSM IP Assets B.V., 6411 TE Heerlen (NL)
(72) Inventor: SALONEN, Jukka, T., FI-70100 Kuopio (FI); FUENTES, Ricardo, 70110 Kuopio (FI); KONTKANEN, Outi, FI-70840 KUOPIO (FI); PIRSKANEN, Mia, FI-70100 KUOPIO (FI); UIMARI, Pekka, FI-70500 Kuopio (FI); AALTO, Juha-Matti, 71800 Siilinjärvi (FI)
(74) Representative: Schwander, Kuno
(86) International application number: PCT/FI2005/050278
(87) International publication number: WO 2006/008342

(56) References cited:
- EP-A2- 0 837 127
- WO-A-2004/048938
- WO-A-2004/092413
- WO-A2-01/78575
- ALTSHULER DAVID ET AL: "The common PPARgamma Pro12Ala polymorphism is associated with decreased risk of type 2 diabetes" NATURE GENETICS, vol. 26, no. 1, September 2000 (2000-09), pages 76-80, XP002556926 ISSN: 1061-4036
- WUYTS W ET AL: "Molecular basis of multiple exostoses: mutations in the EXT1 and EXT2 genes." HUMAN MUTATION 2000, vol. 15, no. 3, 2000, pages 220-227, XP002556927 ISSN: 1059-7794
- DATABASE SNP [Online] NCBI; XP002557027 accession no. rs3814767 -& DATABASE SNP [Online] NCBI; 12 August 2002 (2002-08-12), XP002557028 Database accession no. SS5001179
- DIABETES GENETICS INITIATIVE OF BROAD INSTITUTE OF HARVARD AND MIT, LUND UNIVERSITY, AND NOVARTIS INSTITUTES OF BIOMEDICAL RESEARC: "Genome-wide association analysis identifies loci for type 2 diabetes and triglyceride levels." SCIENCE (NEW YORK, N.Y.) 1 JUN 2007, vol. 316, no. 5829, 1 June 2007 (2007-06-01), pages 1331-1336, XP002556928 ISSN: 1095-9203
- SCOTT LAURA J ET AL: "A genome-wide association study of type 2 diabetes in Finns detects multiple susceptibility variants" SCIENCE, AMERICAN ASSOCIATION FOR THE ADVANCEMENT OF SCIENCE, WASHINGTON, DC; US, vol. 316, no. 5829, 1 June 2007 (2007-06-01), pages 1341-1345, XP002465135 ISSN: 0036-8075
- SLADEK ROBERT ET AL: "A genome-wide association study identifies novel risk loci for type 2 diabetes", NATURE: INTERNATIONAL WEEKLY JOURNAL OF SCIENCE, NATURE PUBLISHING GROUP, UNITED KINGDOM, vol. 445, no. 7130, 22 February 2007 (2007-02-22), pages 881-885, XP002465137, ISSN: 0028-0836, DOI: 10.1038/NATURE05616
- HORIKOSHI M ET AL: "Variations in the HHEX gene are associated with increased risk of type 2 diabetes in the Japanese population", DIABETOLOGIA ; CLINICAL AND EXPERIMENTAL DIABETES AND METABOLISM, SPRINGER, BERLIN, DE, vol. 50, no. 12, 10 October 2007 (2007-10-10), pages 2461-2466, XP019558668, ISSN: 1432-0428, DOI: 10.1007/S00125-007-0827-5

## Description

### BACKGROUND OF THE INVENTION

### Field of the Invention

The present invention relates generally to the field of diagnosing a predisposition for type 2 diabetes mellitus.

### Description of Related Art

The term diabetes mellitus (DM) describes several syndromes of abnormal carbohydrate metabolism that are characterized by hyperglycemia. It is associated with a relative or absolute impairment in insulin secretion, along with varying degrees of peripheral resistance to the action of insulin. The chronic hyperglycemia of diabetes is associated with long-term damage, dysfunction, and failure of various organs, especially the eyes, kidneys, nerves, heart, and blood vessels (ADA, 2003). Type 2 diabetes mellitus (T2D) is characterized by adult onset insulin resistance and a rise in blood sugar concentration.

In 2000, there were approximately 171 million people, worldwide, with diabetes. The number of people with diabetes will expectedly more than double over the next 25 years, to reach a total of 366 million by 2030. Most of this increase will occur as a result of a 150% rise in developing countries. This suggests the role of relatively modem environmental or behavioral risk factors such as high caloric intake or sedentary lifestyle. However, ethnic differences in the incidence and prevalence of T2D and the enrichment of T2D in families suggest heritable risk factors to play a major role.

The two main contributors to the worldwide increase in prevalence of diabetes are population ageing and urbanization, especially in developing countries, with the consequent increase in the prevalence of obesity (WHO/IDF, 2004). Currently more than 1 billion adults are overweight - and at least 300 million of them are clinically obese. Current obesity levels range from below 5% in China, Japan and certain African nations, to over 75% in urban Samoa. The prevalence of obesity is 10-25% in Western Europe and 20-27% in the Americas (WHO, 2004).

In 2000, 3.2 million people died from complications associated with diabetes. Diabetes has become one of the major causes of premature illness and death in most countries, mainly through the increased risk of cardiovascular disease (CVD). Diabetes is a leading cause of blindness, amputation and kidney failure. These complications account for much of the social and financial burden of diabetes (WHO/IDF, 2004).

Because of the chronic nature of T2D, the severity of its complications and the means required to control them, diabetes is a costly disease, not only for the affected individual and his/her family, but also for the health authorities. In the US direct medical and indirect expenditures attributable to diabetes in 2002 were estimated at $132 billion. Direct medical expenditures alone totalled $91.8 billion and comprised $23.2 billion for diabetes care, $24.6 billion for chronic complications attributable to diabetes, and $44.1 billion for excess prevalence of general medical conditions. Attributable indirect expenditures resulting from lost workdays, restricted activity days, mortality, and permanent disability due to diabetes totalled $39.8 billion (ADA, 2003)

According to the new etiologic classification of DM, four categories are differentiated: type 1 diabetes (T1D), type 2 diabetes (T2D), other specific types, and gestational diabetes mellitus (ADA, 2003). In the United States, Canada, and Europe, over 80% of cases of diabetes are due to T2D, 5 to 10% to T1D, and the remainder to other specific causes. In T1D, formerly known as insulin-dependent (IDDM), the pancreas fails to produce the insulin which is essential for survival. This form develops most frequently in children and adolescents, but is being increasingly diagnosed later in life. T2D, formerly named non-insulin-dependent (NIDDM), results from the body's inability to respond properly to the action of insulin produced by the pancreas. T2D occurs most frequently in adults, but is being noted increasingly in adolescents as well (WHO, 2004).

Both T1D and T2D have a complex mode of inheritance, as corroborated by family studies indicating major roles of both inborn susceptibility and the environment. Generally, the sibling of a patient with T1D has a 15-fold higher risk of developing the disease (6%) than does an unrelated individual (0.4%) (Field LL, 2002). In T2D, the absolute risk to siblings is 30%-40%, as compared to a population prevalence of 7%, providing a relative risk to siblings of four to six. In T1D and T2D, rates of concordance are much higher for monozygotic twins as compared to dizygotic twins. Specifically, in T1D, the concordance rate for monozygotic twins is estimated to range from 21%-70%, higher than the 0%-13% range reported for dizygotic twins (Redondo MJ et al, 2001). For T2D, it has been reported that 90% of identical twin pairs were concordant for T2D if followed for long enough (Barnett AH et al, 1981). A concordance rate of 43% in Danish dizygotic twins as compared to 63% in monozygotic twins has also been reported (Poulsen et al, 1999).

Known monogenic forms of diabetes are classified in two categories: genetic defects of the ß-cell and genetic defects in insulin action (ADA, 2003). The diabetes forms associated with monogenetic defects in ß-cell function are frequently characterized by onset of hyperglycemia at an early age (generally before age 25 years). They are referred to as maturity-onset diabetes of the young (MODY) and are characterized by impaired insulin secretion with minimal or no defects in insulin action (Herman WH et al, 1994; Clement K et all, 1996; Byrne MM et al, 1996). They are inherited in an autosomal dominant pattern. Abnormalities at three genetic loci on different chromosomes have been identified to date. The most common form is associated with mutations on chromosome 12 in the locus of a hepatic transcription factor referred to as hepatocyte nuclear factor (HNF)-1α (Vaxillaire M et all, 1995; Yamagata K et al, 1996). A second form is associated with mutations in the locus of the glucokinase gene on chromosome 7p and results in a defective glucokinase molecule (Froguel P et al, 1992; Vionnet N et al, 1992). Glucokinase converts glucose to glucose-6-phosphate, the metabolism of which, in turn, stimulates insulin secretion by the ß-cell. Because of defects in the glucokinase gene, increased plasma levels of glucose are necessary to elicit normal levels of insulin secretion. A third form is associated with a mutation in the HNF-4α gene on chromosome 20q (Bell GI et al, 1991; Yamagata K et al, 1996). HNF-4α is a transcription factor involved in the regulation of the expression of HNF-1α. Point mutations in mitochondrial DNA can cause DM primarily by impairing pancreatic ß-cell function (Reardon W et al, 1992; van den Ouwenland JMW et al, 1992; Kadowaki T et al, 1994). There are unusual causes of diabetes that result from genetically determined abnormalities of insulin action. The metabolic abnormalities associated with mutations of the insulin receptor may range from hyperinsulinemia and modest hyperglycemia to severe diabetes (Kahn CR et al, 1976; Taylor SI, 1992).

Five genome-wide linkage scans have been published for T1D (Davies JL et al, 1994; Hashimoto L et al, 1994; Concannon P et al, 1998; Mein CA et al, 1998; Vaessen N et al, 2002). Also an international cooperative project produced the largest whole-genome linkage scan to date (Cox NJ et al, 2001), which confirmed and extended previous observations. All linkage scans have independently obtained significant evidence for linkage to the Human Leukocyte Antigen (HLA) locus. The association of the insulin VNTR region to T1D was seen only in the cooperative project. Other linkage peaks were found in the chromosome region 2q31-35, which contains the CTLA4 gene, for which further support was obtained by association studies (Nisticò L et al, 1996; Marron MP et al, 1997).

Multiple published genome-wide linkage analyses have searched for regions conferring risk of T2D (Florez JC et al, 2003). In contrast to the HLA region in T1D, no single region has been widely replicated in T2D. Only a few regions have shown significant evidence for linkage in a single scan (LOD score >3.6), or consistent replication across scans. Regions that have shown evidence for linkage in more than one study include chromosomes 1q25.3, 2q37.3, 3p24.1, 3q28, 10q26.13, 12q24.31, and 18p11.22. One of the earliest significant linkage peaks was at chromosome 2q37.3, which led to the identification of CAPN10 gene (Hanis CL et al, 1996; Cox NJ et al, 1999; HorikawaY et al, 2000). However, several subsequent reports have not reproduced these initial results even in closely related populations (Florez JC et al, 2003).

To date, the yield from genome-wide linkage studies of T1D and T2D has been limited. Although many putative localizations have been suggested, fewer are validated by replication across many studies, and there is no clear successful path for progressing from linkage to gene identification.

The failure has in part been due to too small a number of genetic markers used in genome-wide scans (GWS), and in part due to too heterogeneous study populations.
Although many putative gene associations to the common forms of T I D and T2D have been reported, only a handful have been widely replicated. In T1D, there are three convincing associations: the HLA region (Singal DP and Blajcham MA, 1973; Cudworth AG and Woodrow JC, 1974; Nerup JP et al, 1974; Platz et al, 1981; Rotter JI et al, 1983; Tood JA et al, 1987; Sheehy MJ et al, 1989; Todd JA et al, 1989), the insulin VNTR (Bell GI et al, 1984; Hitman GA et al, 1985; Bennett ST and Todd AJ, 1996), and CTLA4 (Nisticò L et al, 1996; Marron MP et al, 1997). These three genes explain about half of the genetic risk of T1D and thus represent an unusual success in identifying the genetic basis of a complex, polygenic disease. In T2D, the association of PPARγ is widely reproduced (Deeb SS et al, 1998; Hara K et al, 2000; Altshuler D et al, 2000; Mori H et al, 2001), and that of KCNJ1 (Hani EH et al, 1998; Gloyn AL et al, 2001; Gloyn AL et al, 2003), ABCC8 (Inoue H, 1996; Hani EH et al, 1997; Hansen T et al, 1998), GCGR (Hager J et al, 1995; Gough SC et al, 1995), GCK (Chiu KC et al, 1992; McCarthy MI et al, 1994; Takekawa K et al, 1994) and SLC2A1 (Li SR et al, 1988; Tao T et al, 1995; Pontiroli AE et al, 1996) have now been seen by multiple groups. Identification of genes causing the major public health problems such as T2D is now enabled by the following recent advances in molecular biology, population genetics and bioinformatics: the availability of new genotyping platforms that will dramatically lower operating cost and increase throughputs; the application of genome scans using dense marker maps (> 100000 markers); data analysis using new powerful statistical methods testing for linkage disequilibrium using haplotype sharing analysis, and the recognition that a smaller number of genetic markers than previously thought is sufficient for genome-wide scans in genetically homogeneous populations.

It is important for the health care system to develop strategies to prevent T2D. Once T2D has manifested clinically, irreversible cell death and tissue damage starts to occur in a number of target tissues such as the arteries, kidneys, nerves and the brain, and the retina of the eye. Unfortunately, the neurons that die cannot be revived or replaced from a stem cell population. Therefore, it is better to prevent T2D from happening in the first place. Although we already know of certain clinical risk factors that increase T2D probability, there is an unmet medical need to define the genetic factors involved in T2D to more precisely define disease risk or susceptibility. There is also a great need for therapeutic agents whose use prevents T2D and diabetic complications.

WO 01/78575 discloses that EXT2 is a marker for osteoporosis or altered bone mineral density.

EP-A-0 837 127 suggest the treatment with EXT2 of patients in need of EXT2.

### SUMMARY OF THE INVENTION

The present invention relates to estimating predisposition of type 2 diabetes as defined in the claims. Discloses is a novel role for exostoses 2 (EXT2) gene and it's encoded proteins and polypeptides. We have mapped a gene conferring susceptibility to T2D to chromosome 11p12-p11 to the EXT2 gene region in the East Finnish population. There are nucleid acid sequence variations in the EXT2 that strongly elevate the probability for T2D in a subject. Further analysis of our results indicated that EXT2 may be associated with the biochemical pathways of T2D.

The first major application involves prediction of those at higher risk of developing a metabolic disease such as T2D. Diagnostic tests that define genetic factors contributing to T2D might be used together with or independent of the known clinical probability factors to define an individual's probability relative to the general population. Better means for identifying those individuals at probability for T2D should lead to better preventive and treatment regimens, including more aggressive management of the current clinical risk factors such as obesity, lack of physical activity, and inflammatory components as reflected by increased C-reactive protein levels or other inflammatory markers. Information on genetic risk may be used by physicians to help convinse particular patients to adjust life style (e.g. to reduce caloric intake, to increase exercise). Disclosed are methods and test kits for determining the presence of a genetic component that doubles an individual's probability for developing T2D. The methods of diagnosing a predisposition to T2D in an individual include detecting the presence of polymorphisms in EXT2 gene, detecting alterations in expression of an EXT2 polypeptide or isoform, such as the presence or relative expression of different splicing variants of EXT2 polypeptides, detecting alterations in biological activity of EXT2 polypeptides as well as detecting alterations in function of metabolic and pathophysiological pathways related to the EXT2 gene. For example, it may be that the expression of certain splice variants of the EXT2 gene or presence of certain SNP markers in the genome of a subject could be used as a diagnostic marker for T2D predisposition. Test kits for estimating susceptibility to T2D in an individual comprise wholly or in part: amplification reagents, detection reagents and interpretation software for computer analysis.

In one aspect, the invention relates to a method as defined in the claims of diagnosing susceptibility to T2D in an individual by screening for the presence of the at-risk haplotypes in the EXT2 gene that are more frequently present in an individual susceptible to T2D, compared to the frequency of its presence in the general population, wherein the presence of an at-risk haplotype is indicative of a susceptibility to T2D.One such at-risk haplotype is haplotype "GGGTG"(or nucleotides from the complementary strand), defined by the SNP markers rs1518820 (G/T) (SEQ ID NO:84), rs1518818 (G/T) (SEQ ID NO:85), rs886196 (A/G) (SEQ ID NO:89), rs2863032 (C/T) (SEQ ID NO:90) and rs3814767 (A/G) (SEQ ID NO:93).

The second major application is the identification of a new pathway involved in T2D. While many have attempted to find genes that are over-expressed or under-expressed in the adipose or muscular tissue taken from T2D, the vast majority of the changes seen compared with tissues taken from non-diabetics are simply a reaction to the underlying process of T2D predisposition and are not the underlying cause. A disease gene with genetic variation that is significantly more common in T2D patients as compared with non-diabetic controls, represents a specifically validated causative step in the pathogenesis of T2D. That is, the uncertainty about whether a gene is causative or simply reactive to the disease process is eliminated. The protein encoded by the EXT2 gene is related to a molecular pathway involved in the biological process of T2D predisposition. Thus the polypeptides encoded by the EXT2 gene or a gene from EXT2 related molecular pathways may represent drug targets that may be selectively modulated by small molecules, proteins, antibodies, or nucleic acid therapies. Such specific information is greatly needed since T2D prevention and treatment is a major unmet medical need that affects i.e.over a million of Americans each year.

Disclosed is an assay for identifying agents that alter (e.g., enhance or inhibit) the EXT2 metabolic activity i.e. EXT2 gene expression, EXT2 biological activity, EXT2 substrate specificity, EXT2 polypeptide primary, secondary or tertiary structure, EXT2 concentration or EXT2 polypeptide degradation. Useful agents include, but are not limited to, binding partners, agonists, antagonists and antibodies of EXT2 polypeptides. Such an assay may also identify agents that alter the relative expression of one or more EXT2 isoforms with respect to other isoforms at either the mRNA level or polypeptide level. For example, a cell, cellular fraction, or solution containing an EXT2 polypeptide or a fragment or derivative thereof, can be contacted with an agent to be tested, and the level of EXT2 polypeptide expression or activity can be assessed. Alternatively, a cell, or cell with recombinant DNA construct with part or all of the EXT2 gene with or without a reporter gene can be used to identify agents that may directly affect transcription i.e. selection of EXT2 transcription start site, splicing pattern and mRNA stability. The effect of an agent on function of EXT2 related metabolic pathways a certain genes of said pathways may be assessed concurrently with the same assays. Resulting information is useful when developing drugs having effect on EXT2 or EXT2 related pathways.

Further disclosed are pharmaceutical compositions comprising nucleic acids polypeptides the invention, and/or the agents that alter activity of EXT2 polypeptide or the function of EXT2 related metabolic pathways. Further disclosed are methods of treating T2D, by administering EXT2 therapeutic agents, such as nucleic acids, polypeptides, the agents that alter activity or quantity of EXT2 polypeptide or the function of EXT2 related metabolic pathways, or compositions comprising the nucleic acids, polypeptides, and/or the agents that alter activity or quantity of EXT2 polypeptide. The pharmaceutical compositions are also useful for preventing the development of T2D in an individual in need thereof by compensating altered expression of EXT2 gene, altered activity of EXT2 polypeptide or altered function of EXT2 related metabolic pathway when compared to a healthy individual. Expression of EXT2 gene, activity of EXT2 polypeptide or function of EXT2 related metabolic pathway can be altered, compared to T2D-free control levels.

A third major application is its use to predict an individual's response to a particular drug, even drugs that do not act on EXT2 gene or polypeptide, but act on EXT2 related pathway. It is a well-known phenomenon that in general, patients do not respond equally to the same drug. Much of the differences in drug response to a given drug is thought to be based on genetic and protein differences among individuals in certain genes and their corresponding pathways. Disclosed is the EXT2 pathway as one key molecular pathway involved in T2D. Some current or future therapeutic agents may be able to affect this pathway directly or indirectly and therefore, be effective in those patients whose T2D probability is in part determined by genetic variations in EXT2 pathway. On the other hand, those same drugs may be less effective or ineffective in those patients who do not have T2D associated variation or haplotype in the EXT2 gene or pathway. Therefore, EXT2 variation or haplotypes may be used as a pharmacogenomic diagnostic to predict drug response and guide choice of therapeutic agent in a given individual.

The disclosure helps meet the unmet medical needs in at least two major ways: 1) it provides a means to define patients at higher risk for T2D than the general population who can be more aggressively managed by their physicians in an effort to prevent T2D and; 2) it defines a drug target that can be used to screen and develop therapeutic agents that can be used to prevent T2D before it happens or prevent complications of T2D in those who have already developed a clinical T2D.

### DETAILED DESCRIPTION OF THE INVENTION

A GWS of T2D was performed using Affymetrix Centurion 120k microarray with almost 100,000 SNP markers as described below. The sample analyzed included 15 unrelated cases and 15 unrelated controls. Haplotype mapping (HPM-G) analysis gave 12 chromosomal regions significantly associated (p-value < 0.001) with T2D (3p26.3, 3p24.2, 3q13.3, 3q22.3, 5q13.3, 5q14.3, 5q23.3, 6p22.2-22.1, 7p22.1, 7q22.1, 8q22.2 and 11p11.2). FM using a total of 17 SNPs (9 SNPs from Affymetrix 120k chip plus 8 additional SNPs from dbSNP) was performed for the most significant region (p-value < 0.0001), the region in chromosome 11p11.2 (bp-location: chr11: 43678152-44345353, bp-extension: 667201). The sample analyzed included 51 unrelated cases and 51 unrelated controls. Point-wise analysis found SNPs rs3814767 (SEQ ID NO:93), rs4379834 (SEQ ID NO:94) and rs962848 (SEQ ID NO:97) significantly associated with T2D (p-value < 0.03). All these SNPs are located in introns of the EXT2 gene. HPM-G analysis found the distribution of the haplotype conformed by the SNPs rs1518820 (SEQ ID NO:84), rs1518818 (SEQ ID NO:85), rs886196 (SEQ ID NO:89), rs2863032 (SEQ ID NO:90) and rs3814767 (SEQ ID NO:93) significantly different between cases and controls (Chi-square value of 18.7, p-value = 0.00002; odds ratio (OR) 27.3, 95% confidence interval (CI) 3.5-214.2), further narrowing the region to chr11: 43837848-44080051 (bp-extension: 242203). Thus, both point-wise and HPM-G analyses showed rs3814767 in EXT2 gene significantly associated with T2D. Results from resequencing showed association of three additional SNP markers, present in EXT2, with increased risk of T2D.

### Possible mechanisms explaining the association between the EXT2 gene and T2D

### EXT2 gene

The EXT2 gene is a known protein coding gene located at 11p12-p11. This gene encodes one of two glycosyltransferases, exostoses 2, involved in the chain elongation step of heparan sulfate biosynthesis. Mutations in this gene cause the type II form of hereditary multiple exostoses, HME. The EXT2 gene belongs to a highly conserved family of genes: EXT1 (8q24.11-q24.13), EXT2 (11p12-p11), EXT3 (19p), EXTL1 (1p36.1), EXTL2 (1p21) and EXTL3 (8p21). A total of 12 mutations in the EXT2 gene have been identified in HME families (Wuyts W et al, 1998, table 1). The gene function has been found involved in the processes of cell growth and/or maintenance, glycosaminoglycan biosynthesis, negative regulation of cell cycle, signal transduction and skeletal development. The gene is expressed in bone, bone marrow, Islets of Langerhans of the pancreas, muscle, liver, kidney, embryonic stem cells, heart, blood vessels, among other tissues and also in multiple tumors and carcinomas

**Table 1: Mutations identified previously in the EXT2 gene**

| | cDNA Change* | Exon or Intron | Protein Change | Type of Mutation |
|---|---|---|---|---|
| 1 | C67T | Exon 2 | Q23X | Nonsense |
| 2 | 77-78insT | Exon 2 | FS Y26 | Frameshift |
| 3 | 449del4 | Exon 2 | FS A150 | Frameshift |
| 4 | C514T | Exon 2 | Q172X | Nonsense |
| 5 | 649-652delT | Exon 4 | FS S218 | Frameshift |
| 6 | C666G | Exon 4 | Y222X | Nonsense |
| 7 | G679A | Exon 4 | D227N | Missense |
| 8 | 812-814delC | Exon 5 | FS A271 | Frameshift |
| 9 | 1173+1G → A | Intron 7 | FS R360 | Splice site |
| 10 | 1173+1G → T | Intron 7 | FS R360 | Splice site |
| 11 | C1201T | Exon 8 | Q401 X | Nonsense |
| 12 | 1263insAT | Exon 8 | FS A422 | Frameshift |

| | | | | |
|---|---|---|---|---|
| *All mutations were numbered uniformly; the adenosine of the start codon was assigned nucleotide position +1 Modified from Wuyts et al, 1998 | | | | |

### Hereditary multiple exostoses

HME, an autosomal dominant bone disorder, is the most common type of benign bone tumor, with an estimated occurrence of 1 in 50,000-100,000 in Western populations. It is characterized by cartilage-capped tumors, known as osteochondromas or exostoses, which develop primarily on the long bones of affected individuals from early childhood until puberty. HME is linked mainly to EXT1 and EXT2, with rare linkage to EXT3. Individuals with HME might have a significantly higher risk than the general population, 0.5-3%, of developing subsequent malignancies such as chondrosarcomas or osteosarcomas (Duncan G et al, 2001). No association between HME and DM has been reported.

### Bone and joint anomalies associated with DM

In adult patients with T1D a moderately reduced bone mineral density (BMD) has been shown in both axial and appendicular skeleton. On the contrary, patients with T2D seem to have higher BMD in respect to healthy control subjects, especially when overweight women are considered. Several mechanisms may decrease BMD, including the increased urinary excretion coupled with the lower intestinal absorption of calcium, the inappropriate homeostatic response in terms of parathyroid hormone secretion, the complex alteration of vitamin D regulation, decreased or increased insulin and insulin-like growth factors concentrations, the effects of the accumulation of glycation endproducts on the bone tissue, and genetic predisposition (Carnevale V et al, 2004).

Diabetic patients (T1D and T2D) have a higher risk for fracture, in particular for hip fracture, the most dangerous complication. This seems to be dependent both on qualitative and quantitative alterations of the bone, as well as on extra-skeletal factors due to the neuropathic and microangiopathic complications of the disease (Espallargues M et al, 2001; Leidig-Bruckner G et al, 2001; Carnevale V et al, 2004). Diffuse idiopathic skeletal hyperostosis (DISH), also known as ankylosing hyperostosis or Forestier's disease, is characterised by new bone formation, particularly in the thoracolumbar spine. Ossification of ligaments and tendons elsewhere may occur, such as the skull, pelvis, heels, or elbows. Estimated prevalence is 13-49% in diabetic patients and 1.6-13% in non-diabetics. Among patients with DISH, 12-80% have diabetes or impaired glucose tolerance. The high prevalence of abnormal glucose tolerance tests in patients with DISH is partly a result of an association with obesity, with 83% of patients being male and 30% obese. A proposed mechanism of causation is the prolonged and high levels of insulin or insulin-like growth factors occurring in diabetic patients, stimulating new bone growth, and may explain the higher prevalence in T1D compared with T2D (ratio 3:1) (Smith LL et al, 2003).

Charcot's disease, or joints, is a result of diabetic peripheral neuropathy affecting up to 35% of patients. A reduction in the normal afferent protective neural impulses, and therefore loss of protection from trauma to the joint leads to progressive, painless joint destruction and bone deformation (exostoses). Charcot's joints are typically seen in patients over the age of 50 who have had diabetes for many years and have neuropathic complications. The joints most commonly affected are weight-bearing joints such as the foot, ankles, and knees; joints such as the hand and wrist are rarely affected (Smith LL et al, 2003).

### Possible mechanisms explaining the link of the EXT2 gene to T2D

In addition to their presumed role as tumor suppressor genes, EXT1 and EXT2 may have roles in modulation of the Hedgehog (Hh) signalling pathway and glycosaminoglycan synthesis. The Hh-family of intercellular signalling proteins (Sonic hedgehog (Shh), Indian hedgehog (Ihh), and Desert hedgehog (Dhh) in Mammals) are key mediators of many fundamental processes in embryonic development (Ingham PW and McMahon AP, 2001). EXT2 gene could exert its influence on type 2 diabetes development in part or entirely already in the embryonic phase. The activities of Hh-signaling proteins are central to the growth, patterning, and morphogenesis of many different regions within the body plans of vertebrates and insects. Hh-signals can act as morphogens in the dose-dependent induction of distinct cell fates within a target field, as mitogens regulating cell proliferation or as inducing factors controlling the form of a developing organ (Ingham PW and McMahon AP, 2001). Heparan sulfate proteoglycans (HSPG) have been implicated in regulating the signalling activities of secreted morphogen molecules including Hh-signalling proteins. HSPG consists of a protein core to which heparan sulfate (HS) and glycosaminoglycan (GAG) chains are attached. The formation of HS GAG chains is catalyzed by glycosyltransferases encoded by members of the EXT gene family (Bellaiche Y et al, 1998; Perrimon N and Bemfield M, 2000; Han C et al, 2004). Hh-signalling is one of the intercellular signals that govern pancreas morphogenesis and cell differentiation (Kim SK and Hebrok M, 2001; Kawahira H et al, 2003), but also continues to signal differentiated beta-cells of the endocrine pancreas in regulating insulin production (Thomas MK et al, 2000). Islet cells differentiate from the epithelial cells of primitive pancreatic ducts during embryogenesis, and can regenerate in response to the loss of islet cells even in adult pancreas (Yamaoka T and Itakura M, 1999). Rat Reg (regenerating) gene was isolated as a gene specifically expressed in regenerating islets (Terazono K et al, 1988). The human regenerating gene, REG1A, was later isolated and characterized (Watanabe T et al, 1990). Rat and human Reg proteins stimulated the replication of pancreatic ß-cells and increased the ß-cell mass in 90% depancreatized rats and in NOD mice, resulting in the amelioration of diabetes (Watanabe T et al, 1994; Gross DJ et al, 1998). Kobayashi et al. (2000) isolated a cDNA for a Reg protein receptor from a rat islet cDNA library. Cells into which the cDNA had been introduced bound Reg protein with high affinity. When the cDNA was introduced into a pancreatic beta-cell line that showed Reg-dependent growth, the transformants exhibited a significant increase in the incorporation of 5'-bromo-2'-deoxyuridine as well as in the cell numbers in response to Reg protein. A homology search revealed that the rat Reg protein receptor cDNA is a homolog of the human EXTL3 gene. The rat and human proteins share 97% sequence identity. These results strongly suggest that the receptor is encoded by the exostoses-like gene and mediates a growth signal of Reg protein for beta-cell regeneration. In humans the Reg gene family is a multigene family grouped into four subclasses, types I, II, III and IV based on the primary structures of the encoded proteins (Zhang YW et al, 2003). Reg family members REG1A, REG1B, REGL and PAP are tandomly clustered on chromosome 2p12 and may have arisen from the same ancestral gene by gene duplication. The REG1A gene encodes a protein secreted by the exocrine pancreas. It is associated with islet cell regeneration and diabetogenesis and may be involved in pancreatic lithogenesis. EXT2 gene through its effect on heparan sulfate biosynthesis affects Hh-signalling, which in turn affects pancreas development and postnatal beta-cells function. This could also explain the frequent finding of BMD anomalies associated with DM. It could also be possible that EXT2 gene (11p12-p11) influences the action of another EXT gene family member, the EXTL3 gene (8p21), which in turn may affect the action of the Reg gene family (2p12) on beta-cell regeneration.

### Representative Target Population

An individual at risk of T2D is an individual who has at least one risk factor, such as obesity, family history of T2D, an at-risk haplotype in one or more T2D probability genes, an at-risk haplotype for the EXT2 gene, a polymorphism in an EXT2 gene, dysregulation of EXT2 expression, altered function of EXT2 related metabolic pathways, elevated body iron stores, an elevated inflammatory marker (e.g., a marker such as C-reactive protein (CRP), serum amyloid A, fibrinogen, tissue necrosis factor-alpha, a soluble vascular cell adhesion molecule (sVCAM), a soluble intervascular adhesion molecule (sICAM), E-selectin, matrix metalloprotease type-1, matrix metalloprotease type-2, matrix metalloprotease type-3, and matrix metalloprotease type-9).

Alternatively, an individual who is at risk of T2D is an individual who has a polymorphism in an EXT2 gene, in which the presence of the polymorphism is indicative of a susceptibility to T2D. The term "gene," as used herein, refers to an entirety containing all regulatory elements located both upstream and downstream as well as within of a polypeptide encoding sequence, 5' and 3' untranslated regions of mRNA and the entire polypeptide encoding sequence including all exon and intron sequences (also alternatively spliced exons and introns) of a gene.

### Assessment for At-Risk Alleles and At-Risk Haplotypes

The genetic markers are particular "alleles" at "polymorphic sites" associated with a disease. A nucleotide position at which more than one sequence is possible in a population, is referred to herein as a "polymorphic site". Where a polymorphic site is a single nucleotide in length, the site is referred to as a SNP. For example, if at a particular chromosomal location, one member of a population has an adenine and another member of the population has a thymine at the same position, then this position is a polymorphic site, and, more specifically, the polymorphic site is a SNP. Polymorphic sites may be several nucleotides in length due to insertions, deletions, conversions or translocations. Each version of the sequence with respect to the polymorphic site is referred to herein as an "allele" of the polymorphic site. Thus, in the previous example, the SNP allows for both an adenine allele and a thymine allele. Typically, a reference nucleotide sequence is referred to for a particular gene. Alleles that differ from the reference are referred to as "variant" alleles. The polypeptide encoded by the reference nucleotide sequence is the "reference" polypeptide with a particular reference amine acid sequence, and polypeptides encoded by variant alleles are referred to as "variant" polypeptides with variant amino acid sequences.

Nucleotide sequence variants can result in changes affecting properties of a polypeptide. These sequence differences, when compared to a reference nucleotide sequence, include insertions, deletions, conversions and substitutions: e.g. an insertion, a deletion or a conversion may result in a frame shift generating an altered polypeptide; a substitution of at least one nucleotide may result in a premature stop codon, aminoacid change or abnormal mRNA splicing; the deletion of several nucleotides, resulting in a deletion of one or more amino acids encoded by the nucleotides; the insertion of several nucleotides, such as by unequal recombination or gene conversion, resulting in an interruption of the coding sequence of a reading frame; duplication of all or a part of a sequence; transposition; or a rearrangement of a nucleotide sequence, as described in detail above. Such sequence changes alter the polypeptide encoded by a disease susceptibility gene. For example, a nucleotide change resulting in a change in polypeptide sequence can alter the physiological properties of a polypeptide dramatically by resulting in altered activity, distribution and stability or otherwise affect on properties of a polypeptide.

Alternatively, nucleotide sequence variants can result in changes affecting transcription of a gene or translation of it's mRNA. A polymorphic site located in a regulatory region of a gene may result in altered transcription of a gene e.g. due to altered tissue specifity, altered transcription rate or altered response to transcription factors. A polymorphic site located in a region corresponding to the mRNA of a gene may result in altered translation of the mRNA e.g. by inducing stable secondary structures to the mRNA and affecting the stability of the mRNA. Such sequence changes may alter the expression of a disease susceptibility gene.

A "haplotype," as described herein, refers to any combination of genetic markers ("alleles") and a haplotype can comprise two or more alleles. As it is recognized by those skilled in the art the same haplotype can be described differently by determining the haplotype defining alleles from different strands e.g. the haplotype rs2221511, rs4940595, rs1522723, rs1395266 (A T C C) described is the same as haplotype rs2221511, rs4940595, rs1522723, rs1395266 (T A G G) in which the alleles are determined from the other strand or haplotype rs2221511, rs4940595, rs 1522723, rs 1395266 (T T C C), in which the first allele is determined from the other strand.

The haplotypes described herein are found more frequently in individuals with a disease than in individuals without the same disease. Therefore, these haplotypes have predictive value for detecting a disease or a susceptibility to a disease in an individual. Therefore, detecting haplotypes can be accomplished by methods known in the art for detecting sequences at polymorphic sites.

It is understood that the T2D associated at-risk alleles and at-risk haplotypes described may be associated with other "polymorphic sites" located in EXT2 gene. These other EXT2 associated polymorphic sites may be either equally useful as genetic markers or even more useful as causative variations explaining the observed association of at-risk alleles and at-risk haplotypes to T2D.

In certain methods described herein, an individual who is at risk for T2D is an individual in whom an at-risk allele or an at-risk haplotype is identified. The at-risk allele or the at-risk haplotype is one that confers a significant risk of T2D. Significance associated with an allele or a haplotype is measured by an odds ratio, e.g. by a percentage. A significant risk is measured as odds ratio of at least about 1.2, including by not limited to: 0.1, 0.2, 0.3, 0.4, 0.5, 0.6, 0.7, 0.8, 0.9, 1.2, 1.3, 1.4, 1.5, 1.6, 1.7, 1.8, 1.9, 2.0, 2.5, 3.0, 4.0, 5.0, 10.0, 15.0, 20.0, 25.0, 30.0 and 40.0. A significant increase or reduction in risk is at least about 20%, including but not limited to about 25%, 30%, 35%, 40%, 45%, 50%, 55%, 60%, 65%, 70%, 75%, 80%, 85%, 90%, 95% and 98%, or at least about 50%. It is understood however, that identifying whether a risk is medically significant may also depend on a variety of factors, including the specific disease, the allele or the haplotype, and often, environmental factors.

### Primers, probes and nucleic acid molecules

"Probes" or "primers" are oligonucleotides that hybridize in a base-specific manner to a complementary strand of nucleic acid molecules. By "base specific manner" is meant that the two sequences must have a degree of nucleotide complementarity sufficient for the primer or probe to hybridize. Accordingly, the primer or probe sequence is not required to be perfectly complementary to the sequence of the template. Non-complementary bases or modified bases can be interspersed into the primer or probe, provided that base substitutions do not inhibit hybridization. The nucleic acid template may also include "non-specific priming sequences" or "nonspecific sequences" to which the primer or probe has varying degrees of complementarity. Such probes and primers include polypeptide nucleic acids (Nielsen PE et al, 1991).

A probe or primer comprises a region of nucleic acid that hybridizes to at least about 15, for example about 20-25, and in certain uses about 40, 50, or 75 consecutive nucleotides of a nucleic acid, such as a nucleic acid comprising a contiguous nucleic acid sequence.

Preferably, a probe or primer comprises 100 or fewer nucleotides, from 6 to 50 nucleotides, for example, from 12 to 30 nucleotides. Preferably , the probe or primer is at least 70% identical to the contiguous nucleic acid sequence or to the complement of the contiguous nucleotide sequence, for example, at least 80% identical, in certain embodiments at least 90% identical, and in other embodiments at least 95% identical, or even capable of selectively hybridizing to the contiguous nucleic acid sequence or to the complement of the contiguous nucleotide sequence. Often, the probe or primer further comprises a label, e.g., radioisotope, fluorescent compound, enzyme, or enzyme co-factor.

Antisense nucleic acid molecules can be designed using the nucleotide sequence of the EXT2 gene and constructed using chemical synthesis and enzymatic ligation reactions using procedures known in the art. For example, an antisense nucleic acid molecule (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Alternatively, the antisense nucleic acid molecule can be produced biologically using an expression vector into which a nucleic acid molecule has been subcloned in an antisense orientation (i.e., RNA transcribed from the inserted nucleic acid molecule will be of an antisense orientation to a target nucleic acid of interest).

The nucleic acid sequences of the EXT2 gene and EXT2 related genes described can also be used to compare with endogenous DNA sequences in patients to identify genetic disorders (e.g., a predisposition for or susceptibility to T2D), and as probes, such as to hybridize and discover related DNA sequences or to subtract out known sequences from a sample. The nucleic acid sequences can further be used to derive primers for genetic fingerprinting, to raise anti-polypeptide antibodies using DNA immunization techniques, and as an antigen to raise anti-DNA antibodies or elicit immune responses. Portions or fragments of the nucleotide sequences identified herein (and the corresponding complete gene sequences) can be used in numerous ways as polynucleotide reagents. For example, these sequences can be used to: (i) map their respective genes on a chromosome; and, thus, locate gene regions associated with genetic disease; (ii) identify an individual from a minute biological sample (tissue typing); and (iii) aid in forensic identification of a biological sample. Additionally, the nucleotide sequences can be used to identify and express recombinant polypeptides for analysis, characterization or therapeutic use, or as markers for tissues in which the corresponding polypeptide is expressed, either constitutively, during tissue differentiation, or in diseased states. The nucleic acid sequences can additionally be used as reagents in the screening and/or diagnostic assays described herein, and can also be included as components of kits (e.g., reagent kits) for use in the screening and/or diagnostic assays described herein.

### Polyclonal and monoclonal antibodies

Polyclonal and/or monoclonal antibodies that specifically bind one form of the gene product but not to the other form of the gene product are also provided. Antibodies are also provided that bind a portion of either the variant or the reference gene product that contains the polymorphic site or sites. The term "antibody" as used herein refers to immunoglobulin molecules and immunologically active portions of immunoglobulin molecules, i.e., molecules that contain an antigen binding site that specifically binds an antigen. A molecule that specifically binds to a polypeptide is a molecule that binds to that polypeptide or a fragment thereof, but does not substantially bind other molecules in a sample, e.g., a biological sample, which naturally contains the polypeptide. Examples of immunologically active portions of immunoglobulin molecules include F(ab) and F(ab') fragments which can be generated by treating the antibody with an enzyme such as pepsin. The term "monoclonal antibody" or "monoclonal antibody composition", as used herein, refers to a population of antibody molecules that contain only one species of an antigen binding site capable of immunoreacting with a particular epitope of a polypeptide. A monoclonal antibody composition thus typically displays a single binding affinity for a particular polypeptide with which it immunoreacts.

Polyclonal antibodies can be prepared as known by those skilled in the art by immunizing a suitable subject with a desired immunogen, e.g., polypeptide disclosed herein or fragment thereof. The antibody titer in the immunized subject can be monitored over time by standard techniques, such as with an enzyme linked immunosorbent assay (ELISA) using immobilized polypeptide. If desired, the antibody molecules directed against the polypeptide can be isolated from the mammal (e.g., from the blood) and further purified by well-known techniques, such as protein A chromatography to obtain the IgG fraction. At an appropriate time after immunization, e.g., when the antibody titers are highest, antibody-producing cells can be obtained from the subject and used to prepare monoclonal antibodies by standard techniques, such as the hybridoma technique (Kohler G and Milstein C, 1975), the human B cell hybridoma technique (Kozbor D et al, 1982), the EBV-hybridoma technique (Cole SP et al, 1994), or trioma techniques (Hering S et al, 1988). To produce a hybridoma an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds a polypeptide disclosed herein.

Any of the many well known protocols used for fusing lymphocytes and immortalized cell lines can be applied for the purpose of generating a monoclonal antibody to a polypeptide (Bierer B et al, 2002). Moreover, the ordinarily skilled worker will appreciate that there are many variations of such methods that also would be useful. Alternative to preparing monoclonal antibody-secreting hybridomas, a monoclonal antibody to a polypeptide can be identified and isolated by screening a recombinant combinatorial immunoglobulin library (e.g., an antibody phage display library) with the polypeptide to thereby isolate immunoglobulin library members that bind the polypeptide (Hayashi N et al, 1995; Hay BN et al, 1992; Huse WD et al, 1989; Griffiths AD et al, 1993). Kits for generating and screening phage display libraries are commercially available.

Additionally, recombinant antibodies, such as chimeric and humanized monoclonal antibodies, comprising both human and non-human portions, can be made using standard recombinant DNA techniques. Such chimeric and humanized monoclonal antibodies can be produced by recombinant DNA techniques known in the art.

In general, antibodies (e.g., a monoclonal antibody) can be used to isolate a polypeptide by standard techniques, such as affinity chromatography or immunoprecipitation. A polypeptide-specific antibody can facilitate the purification of natural polypeptide from cells and of recombinantly produced polypeptide expressed in host cells. Moreover, an antibody specific for a polypeptide can be used to detect the polypeptide (e.g., in a cellular lysate, cell supernatant, or tissue sample) in order to evaluate the abundance and pattern of expression of the polypeptide. Antibodies can be used diagnostically to monitor protein levels in tissue such as blood as part of a test predicting the susceptibility to T2D or as part of a clinical testing procedure, e.g., to, for example, determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### Diagnostic Assays

The probes, primers and antibodies described herein can be used in methods of diagnosis of T2D or diagnosis of a susceptibility to T2D, as well as in kits useful for diagnosis of T2D or susceptibility to T2D or to a disease or condition associated with T2D. Diagnosis of T2D or susceptibility to T2D (or diagnosis of or susceptibility to a disease or condition associated with T2D), may be made by detecting one or several of at-risk alleles or at-risk haplotypes or a combination of at-risk alleles and at-risk haplotypes described in the subject's nucleic acid as described herein.

Diagnosis of T2D or susceptibility to T2D (or diagnosis of or susceptibility to a disease or condition associated with T2D), may be made by detecting one or several of polymorphic sites which are associated with at-risk alleles or/and at-risk haplotypes described in the subject's nucleic acid. Diagnostically the most useful polymorphic sites are those altering the polypeptide structure of an T2D associated gene due to a frame shift; due to a premature stop codon, due to an aminoacid change or due to abnormal mRNA splicing. Nucleotide changes resulting in a change in polypeptide sequence in many case alter the physiological properties of a polypeptide by resulting in altered activity, distribution and stability or otherwise affect on properties of a polypeptide. Other diagnostically useful polymorphic sites are those affecting transcription of an T2D associated gene or translation of it's mRNA due to altered tissue specifity, due to altered transcription rate, due to altered response to physiological status, due to altered translation efficiency of the mRNA and due to altered stability of the mRNA. The presence of nucleotide sequence variants altering the polypeptide structure of T2D associated genes or altering the expression of T2D associated genes is diagnostic for susceptibility to T2D.

For diagnostic applications, there may be polymorphisms informative for prediction of disease risk that are in linkage disequilibrium with the functional polymorphism. Such a functional polymorphism may alter splicing sites, affect the stability or transport of mRNA, or otherwise affect the transcription or translation of the nucleic acid. The presence of nucleotide sequence variants associated with functional polymorphism is diagnostic for susceptibility to T2D. While we have genotyped and included a limited number of example SNP markers in the experimental section, any functional, regulatory or other mutation or alteration described above in the EXT2 gene is expected to predict the risk of T2D.

In diagnostic assays determination of the nucleotides present in one or several of the T2D associated SNP markers, as well as polymorphic sites associated with T2D associated SNP markers, in an individual's nucleic acid can be done by any method or technique which can accurately determine nucleotides present in a polymorphic site. Numerous suitable methods have been described in the art (Kwok P-Y, 2001; Syvänen A-C, 2001), these methods include, but are not limited to, hybridization assays, ligation assays, primer extension assays, enzymatic cleavage assays, chemical cleavage assays and any combinations of these assays. The assays may or may not include PCR, solid phase step, modified oligonucleotides, labeled probes or labeled nucleotides and the assay may be multiplex or singleplex. As it is obvious in the art the nucleotides present in polymorphic site can be determined from one nucleic acid strand or from both strands.

Disclosed is that diagnosis of a susceptibility to T2D can also be made by examining transcription of the EXT2 gene. Alterations in transcription can be analysed by a variety of methods as described in the art, including e.g. hybridization methods, enzymatic cleavage assays, RT-PCR assays and microarrays. A test sample from an individual is collected and the alterations in the transcription of the EXT2 gene are assessed from the RNA present in the sample. Altered transcription is diagnostic for a susceptibility to T2D.

Disclosed is that diagnosis of a susceptibility to T2D can also be made by examining expression and/or structure and/or function of EXT2 polypeptides. A test sample from an individual is assessed for the presence of an alteration in the expression and/or an alteration in structure and/or function of the polypeptide encoded by the EXT2 gene, or for the presence of a particular polypeptide variant (e.g., an isoform) encoded by the EXT2 gene. An alteration in expression of a polypeptide encoded by the EXT gene can be, for example, an alteration in the quantitative polypeptide expression (i.e., the amount of polypeptide produced); an alteration in the structure and/or function of a polypeptide encoded by the EXT2 gene is an alteration in the qualitative polypeptide expression (e.g., expression of a mutant EXT2 polypeptide or of a different splicing variant or isoform). Detection of a particular splicing variant encoded by the EXT2 gene, or a particular pattern of splicing variants makes possible diagnosis of the disease or condition associated with T2D or a susceptibility to a disease or condition associated with T2D is disclosed.

Alterations in expression and/or structure and/or function of EXT2 polypeptides can be determined by various methods known in the art e.g. by assays based on chromatography, spectroscopy, colorimetry, electrophoresis, isoelectric focusing, specific cleavage, immunologic techniques and measurement of biological activity as well as combinations of different assays. An "alteration" in the polypeptide expression or composition, as used herein, refers to an alteration in expression or composition in a test sample, as compared with the expression or composition of polypeptide by the EXT2 gene in a control sample. A control sample is a sample that corresponds to the test sample (e.g., is from the same type of cells), and is from an individual who is not affected by T2D. An alteration in the expression or composition of the polypeptide in the test sample, as compared with the control sample, is indicative of a susceptibility to T2D.

Western blotting analysis, using an antibody as described above that specifically binds to a polypeptide encoded by a mutant EXT2 gene, or an antibody that specifically binds to a polypeptide encoded by a non-mutant gene, or an antibody that specifically binds to a particular splicing variant encoded by the EXT2 gene, can be used to identify the presence in a test sample of a particular splicing variant or isoform, or of a polypeptide encoded by a polymorphic or mutant EXT2 gene, or the absence in a test sample of a particular splicing variant or isoform, or of a polypeptide encoded by a non-polymorphic or non-mutant gene. The presence of a polypeptide encoded by a polymorphic or mutant gene, or the absence of a polypeptide encoded by a non-polymorphic or non-mutant gene, is diagnostic for a susceptibility to T2D, as is the presence (or absence) of particular splicing variants encoded by the EXT2 gene.

Disclosed is that the level or amount of polypeptide encoded by the EXT2 gene in a test sample is compared with the level or amount of the polypeptide encoded by the EXT2 gene in a control sample. A level or amount of the polypeptide in the test sample that is higher or lower than the level or amount of the polypeptide in the control sample, such that the difference is statistically significant, is indicative of an alteration in the expression of the polypeptide encoded by the EXT2 gene, and is diagnostic for a susceptibility to T2D. Alternatively, the composition of the polypeptide encoded by the EXT2 gene in a test sample is compared with the composition of the polypeptide encoded by the EXT2 gene in a control sample (e.g., the presence of different splicing variants). A difference in the composition of the polypeptide in the test sample, as compared with the composition of the polypeptide in the control sample, is diagnostic for a susceptibility to T2D. Both the level or amount and the composition of the polypeptide may be assessed in the test sample and in the control sample. A difference in the amount or level of the polypeptide in the test sample, compared to the control sample; a difference in composition in the test sample, compared to the control sample; or both a difference in the amount or level, and a difference in the composition, is indicative of a susceptibility to T2D.

Dislcosed is that assessment of the splicing variant or isoform(s) of a polypeptide encoded by a polymorphic or mutant EXT2 gene can be performed. The assessment can be performed directly (e.g., by examining the polypeptide itself), or indirectly (e.g., by examining the mRNA encoding the polypeptide, such as through mRNA profiling). For example, probes or primers as described herein can be used to determine which splicing variants or isoforms are encoded by EXT2 gene mRNA, using standard methods.

The presence in a test sample of a particular splicing variant(s) or isoform(s) associated with T2D or risk of T2D, or the absence in a test sample of a particular splicing variant(s) or isoform(s) not associated with T2D or risk of T2D, is diagnostic for a disease or condition associated with the EXT2 gene or a susceptibility to a disease or condition associated with the EXT2 gene. Similarly, the absence in a test sample of a particular splicing variant(s) or isoform(s) associated with T2D or risk of T2D, or the presence in a test sample of a particular splicing variant(s) or isoform(s) not associated with T2D or risk of T2D, is diagnostic for the absence of disease or condition associated with the EXT2 gene or a susceptibility to a disease or condition associated with the EXT2 gene.

The invention pertains to a method for the diagnosis and identification of susceptibility to T2D in an individual, by identifying an at-risk allele or an at-risk haplotype in the EXT2 gene as defined in the claims. In one embodiment, the at-risk allele or the at-risk haplotype is an allele or a haplotype for which the presence of the haplotype increases the risk of T2D significantly. Although it is to be understood that identifying whether a risk is significant may depend on a variety of factors, including the specific disease, the haplotype, and often, environmental factors, the significance may be measured by an odds ratio or a percentage. The significance may be measured by a percentage, e.g. a significant risk is measured as an odds ratio of 0.8 or less or at least about 1.2, including by not limited to: 0.1, 0.2, 0.3, 0.4, 0.5, 0.6, 0.7, 0.8, 0.9, 1.2, 1.3, 1.4, 1.5, 1.6, 1.7, 1.8, 1.9, 2.0, 2.5, 3.0, 4.0, 5.0, 10.0, 15.0, 20.0, 25.0, 30.0 and 40.0, e.g. an odds ratio of at least 1.2 is significant or an odds ratio of at least about 1.5 is significant, or a significant increase or decrease in risk is at least about 1.7. A significant increase in risk is at least about 20%, including but not limited to about 25%, 30%, 35%, 40%, 45%, 50%, 55%, 60%, 65%, 70%, 75%, 80%, 85%, 90%, 95% and 98%, e.g. a significant increase or reduction in risk is at least about 50%. It is understood however, that identifying whether a risk is medically significant may also depend on a variety of factors, including the specific disease, the allele or the haplotype, and often, environmental factors.

Disclosed are methods of diagnosing T2D or a susceptibility to T2D in an individual, comprising screening for an at-risk haplotype in the EXT2 gene that is more frequently present in an individual susceptible to T2D (affected), compared to the frequency of its presence in a healthy individual (control), wherein the presence of the haplotype is indicative of T2D or susceptibility to T2D.

Kits (e.g., reagent kits) useful in the methods of diagnosis comprise components useful in any of the methods described herein, including for example, PCR primers, hybridization probes or primers as described herein (e.g., labeled probes or primers), reagents for genotyping SNP markers, reagents for detection of labeled molecules, restriction enzymes (e.g., for RFLP analysis), allele-specific oligonucleotides, DNA polymerases, RNA polymerases, marker enzymes, antibodies which bind to altered or to non-altered (native) EXT2 polypeptide, means for amplification of nucleic acid fragments from the EXT2 gene, or means for analyzing the nucleic acid sequence of the EXT2 gene or for analyzing the amino acid sequence of EXT2 polypeptides, etc. A kit for diagnosing susceptibility to T2D can comprise primers for nucleic acid amplification of a region in the EXT2 gene comprising an at-risk haplotype that is more frequently present in an individual susceptible to T2D. The primers can be designed using portions of the nucleic acids flanking SNPs that are indicative of T2D.

This disclosure invention is based on the principle that one or a small number of genotypings are performed, and the mutations to be typed are selected on the basis of their ability to predict T2D. For this reason any method to genotype mutations in a genomic DNA sample can be used. If non-parallel methods such as real-time PCR are used, the typings are done in a row.

The PCR reactions may be multiplexed or carried out separately in a row or in parallel aliquots.

Thus, the detection method may further comprise a step of combining information concerning age, gender, the family history of obesity and diabetes, waist-to-hip circumference ratio (cm/cm), and the medical history concerning diabetes of the subject with the results obtained from step b) of the method (see claim 1) for confirming the indication obtained from the detection step. The detection method may also further comprise a step determining blood, serum or plasma fibrinogen, ferritin, transferrin receptor, C-reactive protein and insulin concentration from the subject.

The score that predicts the probability of T2D may be calculated using a multivariate failure time model or a logistic regression equation. The results from the furher steps of the method as described above render possible a step of calculating the probability of T2D using a logistic regression equation as follows. Probability of an T2D = 1/[1 + e (-(-a + Σ(bi*Xi))], where e is Napier's constant, Xi are variables related to the T2D, bi are coefficients of these variables in the logistic function, and a is the constant term in the logistic function, and wherein a and bi are preferably determined in the population in which the method is to be used, and Xi are preferably selected among the variables that have been measured in the population in which the method is to be used. Preferable values for bᵢ are between -20 and 20; and for i between 0 (none) and 100,000. A negative coefficient bᵢ implies that the marker is risk-reducing and a positive that the marker is risk-increasing. Xi are binary variables that can have values or are coded as 0 (zero) or 1 (one) such as SNP markers. The model may additionally include any interaction (product) or terms of any variables Xi, e.g. biXi. An algorithm is developed for combining the information to yield a simple prediction of T2D as percentage of risk in one year, two years, five years, 10 years or 20 years. Alternative statistical models are failure-time models such as the Cox's proportional hazards' model, other iterative models and neural networking models.

### Monitoring Progress of Treatment

Disclosed are methods of monitoring the effectiveness of treatment on the regulation of expression (e.g., relative or absolute expression) of EXT2 at the RNA or protein level or its enzymatic activity. EXT2 message or protein or enzymatic activity can be measured in a sample of peripheral blood or cells derived therefrom. An assessment of the levels of expression or activity can be made before and during treatment with EXT2 therapeutic agents.

For example, an individual who is a member of the target population can be assessed for response to treatment with an EXT2 inhibitor, by examining EXT2 activity or absolute and/or relative levels of EXT2 protein or mRNA isoforms in peripheral blood in general or specific cell subfractions or combination of cell subfractions. In addition, variation such as haplotypes or mutations within or near (within 50 to 200 kb) of the EXT2 gene may be used to identify individuals who are at higher risk for T2D to increase the power and efficiency of clinical trials for pharmaceutical agents to prevent or treat T2D or its complications. The haplotypes and other variations may be used to exclude or fractionate patients in a clinical trial who are likely to have non-EXT2 pathway involvement in their T2D in order to enrich patients who have EXT2-related pathways involved and boost the power and sensitivity of the clinical trial. Such variation may be used as a pharmacogenomic test to guide selection of pharmaceutical agents for individuals

### Screening Assays and Agents Identified Thereby

Disclosed are methods for identifying agents (e.g., fusion proteins, polypeptides, peptidomimetics, prodrugs, receptors, binding agents, antibodies, small molecules or other drugs, or ribozymes) that alter (e.g., increase or decrease) the activity of the polypeptides described herein, or which otherwise interact with the polypeptides herein. For example, such agents can be agents which bind to polypeptides described herein (e.g., EXT2 binding agents); which have a stimulatory or inhibitory effect on, for example, activity of polypeptides; or which change (e.g., enhance or inhibit) the ability of the polypeptides to interact with EXT2 binding agents (e.g., receptors or other binding agents); or which alter posttranslational processing of the EXT2 polypeptide (e.g., agents that alter proteolytic processing to direct the polypeptide from where it is normally synthesized to another location in the cell, such as the cell surface); agents that alter proteolytic processing such that more polypeptide is released from the cell, etc.

Disclosed are assays for screening candidate or test agents that bind to or modulate the activity of polypeptides described herein (or biologically active portion(s) thereof), as well as agents identifiable by the assays. Test agents can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to polypeptide libraries, while the other four approaches are applicable to polypeptide, non-peptide oligomer or small molecule libraries of compounds (Lam KS, 1997).

In order to identity agents which alter the activity of an EXT2 polypeptide, a cell, cell lysate, or solution containing or expressing an EXT2 polypeptide or another splicing variant encoded by EXT2), or a fragment or derivative thereof (as described above), can be contacted with an agent to be tested; alternatively, the polypeptide can be contacted directly with the agent to be tested. The level (amount) of EXT2 activity is assessed (e.g., the level (amount) of EXT2 activity is measured, either directly or indirectly), and is compared with the level of activity in a control (i.e., the level of activity of the EXT2 polypeptide or active fragment or derivative thereof in the absence of the agent to be tested). If the level of the activity in the presence of the agent differs, by an amount that is statistically significant, from the level of the activity in the absence of the agent, then the agent is an agent that alters the activity of EXT2 polypeptide. An increase in the level of EXT2 activity relative to level of the control, indicates that the agent is an agent that enhances (is an agonist of) EXT2 activity. Similarly, a decrease in the level of EXT2 activity relative to level of the control, indicates that the agent is an agent that inhibits (is an antagonist of) EXT2 activity. The level of activity of an EXT2 polypeptide or derivative or fragment thereof in the presence of the agent to be tested, is compared with a control level that has previously been established. A level of the activity in the presence of the agent that differs from the control level by an amount that is statistically significant indicates that the agent alters EXT2 activity.

Disclosed are assays for identifying agents which alter the expression identifying the of the EXT2 gene (e.g., antisense nucleic acids, fusion proteins, polypeptides, peptidomimetics, prodrugs, receptors, binding agents, antibodies, small molecules or other drugs, or ribozymes) which alter (e.g., increase or decrease) expression (e.g., transcription or translation) of the gene or which otherwise interact with the nucleic acids described herein, as well as agents identifiable by the assays. For example, a solution containing a nucleic acid encoding EXT2 polypeptide (e.g., EXT2 gene) can be contacted with an agent to be tested. The solution can comprise, for example, cells containing the nucleic acid or cell lysate containing the nucleic acid; alternatively, the solution can be another solution that comprises elements necessary for transcription/translation of the nucleic acid. Cells not suspended in solution can also be employed, if desired. The level and/or pattern of EXT2 expression (e.g., the level and/or pattern of mRNA or of protein expressed, such as the level and/or pattern of different splicing variants) is assessed, and is compared with the level and/or pattern of expression in a control (i.e., the level and/or pattern of the EXT2 expression in the absence of the agent to be tested). If the level and/or pattern in the presence of the agent differs, by an amount or in a manner that is statistically significant, from the level and/or pattern in the absence of the agent, then the agent is an agent that alters the expression of EXT2. Enhancement of EXT2 expression indicates that the agent is an agonist of EXT2 activity. Similarly, inhibition of EXT2 expression indicates that the agent is an antagonist of EXT2 activity. The level and/or pattern of EXT2 polypeptide(s) (e.g., different splicing variants) in the presence of the agent to be tested, is compared with a control level and/or pattern that has previously been established A level and/or pattern in the presence of the agent that differs from the control level and/or pattern by an amount or in a manner that is statistically significant indicates that the agent alters EXT2 expression.

Agents which alter the expression of the EXT2 gene or which otherwise interact with the nucleic acids described herein, can be identified using a cell, cell lysate, or solution containing a nucleic acid encoding the promoter region of the EXT2 gene operably linked to a reporter gene. After contact with an agent to be tested, the level of expression of the reporter gene (e.g. the level of mRNA or of protein expressed) is assessed, and is compared with the level of expression in a control (i.e., the level of the expression of the reporter gene in the absence of the agent to be tested). If the level in the presence of the agent differs, by an amount or in a manner that is statistically significant, from the level in the absence of the agent, then the agent is an agent that alters the expression of EXT2, as indicated by its ability to alter expression of a gene that is operably linked to the EXT2 gene promoter. Enhancement of the expression of the reporter indicates that the agent is an agonist of EXT2 activity. Similarly, inhibition of the expression of the reporter indicates that the agent is an antagonist of EXT2 activity. The level of expression of the reporter in the presence of the agent to be tested is compared with a control level that has previously been established. A level in the presence of the agent that differs from the control level by an amount or in a manner that is statistically significant indicates that the agent alters EXT2 expression.

Agents which alter the amounts of different splicing variants encoded by EXT2 (e.g., an agent which enhances activity of a first splicing variant, and which inhibits activity of a second splicing variant), as well as agents which are agonists of activity of a first splicing variant and antagonists of activity of a second splicing variant, can easily be identified using these methods described above.

Assays can be used to assess the impact of a test agent on the activity of a polypeptide in relation to an EXT2 binding agent. For example, a cell that expresses a compound that interacts with EXT2 (herein referred to as an "EXT2 binding agent", which can be a polypeptide or other molecule that interacts with EXT2, such as a receptor) is contacted with EXT2 in the presence of a test agent, and the ability of the test agent to alter the interaction between EXT2 and the EXT2 binding agent is determined. Alternatively, a cell lysate or a solution containing the EXT2 binding agent, can be used. An agent which binds to EXT2 or the EXT2 binding agent can alter the interaction by interfering with, or enhancing the ability of EXT2 to bind to, associate with, or otherwise interact with the EXT2 binding agent. Determining the ability of the test agent to bind to EXT2 or an EXT2 binding agent can be accomplished, for example, by coupling the test agent with a radioisotope or enzymatic label such that binding of the test agent to the polypeptide can be determined by detecting the labeled with 125I, sup35S, sup14C or sup3H, either directly or indirectly, and the radioisotope detected by direct counting of radioemmission or by scintillation counting. Alternatively, test agents can be enzymatically labeled with, for example, horseradish peroxidase, alkaline phosphatase, or luciferase, and the enzymatic label detected by determination of conversion of an appropriate substrate to product. The ability of a test agent to interact with the polypeptide may be determined without the labeling of any of the interactants. For example, a microphysiometer can be used to detect the interaction of a test agent with EXT2 or an EXT2 binding agent without the labeling of either the test agent, EXT2, or the EXT2 binding agent (McConnell HM et al, 1992). As used herein, a "microphysiometer", e.g. Cytosensor ™ is an analytical instrument that measures the rate at which a cell acidifies its environment using a light-addressable potentiometric sensor (LAPS). Changes in this acidification rate can be used as an indicator of the interaction between ligand and polypeptide. See the Examples Section for a discussion of known EXT2 binding partners. Thus, these receptors can be used to screen for compounds that are EXT2 receptor agonists for use in treating T2D or EXT2 receptor antagonists for studying T2D. The linkage data provided herein, for the first time, provides such connection to T2D. Drugs could be designed to regulate EXT2 receptor activation that in turn can be used to regulate signaling pathways and transcription events of genes downstream, such as Cbfa1.

Assays can be used to identify polypeptides that interact with one or more EXT2 polypeptides, as described herein. For example, a yeast two-hybrid system such as that described by (Fields S and Song O, 1989) can be used to identify polypeptides that interact with one or more EXT2 polypeptides. In such a yeast two-hybrid system, vectors are constructed based on the flexibility of a transcription factor that has two functional domains (a DNA binding domain and a transcription activation domain). If the two domains are separated but fused to two different proteins that interact with one another, transcriptional activation can be achieved, and transcription of specific markers (e.g., nutritional markers such as His and Ade, or color markers such as lacZ) can be used to identify the presence of interaction and transcriptional activation. For example, a first vector is used which includes a nucleic acid encoding a DNA binding domain and also an EXT2 polypeptide, splicing variant, fragment or derivative thereof, and a second vector is used which includes a nucleic acid encoding a transcription activation domain and also a nucleic acid encoding a polypeptide which potentially may interact with the EXT2 polypeptide, splicing variant, or fragment or derivative thereof (e.g., a EXT2 polypeptide binding agent or receptor). Incubation of yeast containing the first vector and the second vector under appropriate conditions allows identification of colonies which express the markers of interest. These colonies can be examined to identify the polypeptide(s) that interact with the EXT2 polypeptide or fragment or derivative thereof. Such polypeptides may be useful as agents that alter the activity of expression of an EXT2 polypeptide, as described above.

In the above assay methods, it may be desirable to immobilize either EXT2, the EXT2 binding agent, or other components of the assay on a solid support, in order to facilitate separation of complexed from uncomplexed forms of one or both of the polypeptides, as well as to accommodate automation of the assay. Binding of a test agent to the polypeptide, or interaction of the polypeptide with a binding agent in the presence and absence of a test agent, can be accomplished in any vessel suitable for containing the reactants. Examples of such vessels include microtitre plates, test tubes, and micro-centrifuge tubes. A fusion protein (e.g., a glutathione-S-transferase fusion protein) can be provided which adds a domain that allows EXT2 or an EXT2 binding agent to be bound to a matrix or other solid support.

Modulators of expression of nucleic acid molecules may be identified in a method wherein a cell, cell lysate, or solution containing a nucleic acid encoding EXT2 is contacted with a test agent and the expression of appropriate mRNA or polypeptide (e.g., splicing variant(s)) in the cell, cell lysate, or solution, is determined. The level of expression of appropriate mRNA or polypeptide(s) in the presence of the test agent is compared to the level of expression of mRNA or polypeptide(s) in the absence of the test agent. The test agent can then be identified as a modulator of expression based on this comparison. For example, when expression of mRNA or polypeptide is statistically significantly greater in the presence of the test agent than in its absence, the test agent is identified as a stimulator or enhancer of the mRNA or polypeptide expression. Alternatively, when expression of the mRNA or polypeptide is statistically significantly less in the presence of the test agent than in its absence, the test agent is identified as an inhibitor of the mRNA or polypeptide expression. The level of mRNA or polypeptide expression in the cells can be determined by methods described herein for detecting mRNA or polypeptide.

Disclosed are novel agents identified by the above-described screening assays. Accordingly, an agent identified can be used as described herein in an appropriate animal model. For example, an agent identified as described herein (e.g., a test agent that is a modulating agent, an antisense nucleic acid molecule, a specific antibody, or a polypeptide-binding agent) can be used in an animal model to determine the efficacy, toxicity, or side effects of treatment with such an agent. Alternatively, an agent identified as described herein can be used in an animal model to determine the mechanism of action of such an agent. Furthermore, uses of novel agents identified by the above-described screening assays for treatments as described herein are disclosed. In addition, an agent identified as described herein can be used to alter activity of a polypeptide encoded by EXT2, or to alter expression of EXT2, by contacting the polypeptide or the gene (or contacting a cell comprising the polypeptide or the gene) with the agent identified as described herein.

### Methods of Therapy

Disclosed are methods of treatment (prophylactic and/or therapeutic) for T2D or a susceptibility to T2D, such as individuals in the target populations described herein, using an EXT2 therapeutic agent. An "EXT2 therapeutic agent" is an agent that alters (e.g., enhances or inhibits) EXT2 polypeptide (enzymatic activity or quantity) and/or EXT2 gene expression, as described herein (e.g., an EXT2 agonist or antagonist), or alters function of an EXT2 related metabolic pathway. EXT2 therapeutic agents can alter EXT2 polypeptide activity or nucleic acid expression by a variety of means, such as, for example, by providing additional EXT2 polypeptide or by upregulating the transcription or translation of the EXT2 gene; by altering posttranslational processing of the EXT2 polypeptide; by altering transcription of EXT2 splicing variants; or by interfering with EXT2 polypeptide activity (e.g., by binding to an EXT2 polypeptide); or by downregulating the transcription or translation of the EXT2 gene, or by inhibiting or enhancing the elimination of EXT2 polypeptide.

In particular, the disclosure relates to methods of treatment for T2D or susceptibility to T2D (for example, for individuals in an at-risk population such as those described herein); as well as to methods of treatment for macrovascular complications of T2D including but not limited to myocardial infarction, angina pectoris, atherosclerosis, acute coronary syndrome (e.g., unstable angina, non-ST-elevation myocardial infarction (NSTEMI) or ST-elevation myocardial infarction (STEMI)), peripheral arterial occlusive disease, cerebrovascular stroke, congestive heart failure and cardiac hypertrophy, or microvascular complications including but not limited to retinopathy, neuropathy and nephropathy.

*Representative EXT2 therapeutic agents include the following:*
nucleic acids or fragments or derivatives related to the EXT2 gene, particularly nucleotides encoding EXT2 polypeptides and vectors comprising such nucleic acids (e.g., a gene, cDNA, and/or mRNA, double-stranded interfering RNA, a nucleic acid encoding an EXT2 polypeptide or active fragment or derivative thereof;
EXT2 polypeptides including splicing variants of EXT2, or fragments or derivatives thereof;
other polypeptides (e.g., EXT2 receptors); EXT2 binding agents; peptidomimetics; fusion proteins or prodrugs thereof, antibodies (e.g., an antibody to a mutant EXT2 polypeptide, or an antibody to a non-mutant EXT2 polypeptide, or an antibody to a particular splicing variant encoded by EXT2, as described above); ribozymes; other small molecules;
and other agents that alter (e.g., inhibit or antagonize) EXT2 gene expression or polypeptide activity, or that regulate transcription of EXT2 splicing variants (e.g., agents that affect which splicing variants are expressed, or that affect the amount of each splicing variant that is expressed).

More than one EXT2 therapeutic agent can be used concurrently, if desired.

The EXT2 therapeutic agent that is a nucleic acid is used in the treatment of T2D. The term, "treatment" as used herein, refers not only to ameliorating symptoms associated with the disease, but also preventing or delaying the onset of the disease, and also lessening the severity or frequency of symptoms of the disease, preventing or delaying the occurrence of a second episode of the disease or condition; and/or also lessening the severity or frequency of symptoms of the disease or condition. In the case of atherosclerosis, "treatment" also refers to a minimization or reversal of the development of plaques. The therapy is designed to alter (e.g., inhibit or enhance), replace or supplement activity of an EXT2 polypeptide in an individual. For example, an EXT2 therapeutic agent can be administered in order to upregulate or increase the expression or availability of the EXT2 gene or of specific splicing variants of EXT2, or, conversely, to downregulate or decrease the expression or availability of the EXT2 gene or specific splicing variants of EXT2. Upregulation or increasing expression or availability of a native EXT2 gene or of a particular splicing variant could interfere with or compensate for the expression or activity of a defective gene or another splicing variant; downregulation or decreasing expression or availability of a native EXT2 gene or of a particular splicing variant could minimize the expression or activity of a defective gene or the particular splicing variant and thereby minimize the impact of the defective gene or the particular splicing variant.

The EXT2 therapeutic agent(s) are administered in a therapeutically effective amount (i.e., an amount that is sufficient to treat the disease, such as by ameliorating symptoms associated with the disease, preventing or delaying the onset of the disease, and/or also lessening the severity or frequency of symptoms of the disease). The amount which will be therapeutically effective in the treatment of a particular individual's disorder or condition will depend on the symptoms and severity of the disease, and can be determined by standard clinical techniques. In addition, in vitro or in vivo assays may optionally be employed to help identify optimal dosage ranges. The precise dose to be employed in the formulation will also depend on the route of administration, and the seriousness of the disease or disorder, and should be decided according to the judgment of a practitioner and each patient's circumstances. Effective doses may be extrapolated from dose-response curves derived from in vitro or animal model test systems.

EXT2 or a cDNA encoding the EXT2 polypeptide, either by itself or included within a vector, can be introduced into cells (either in vitro or in vivo) such that the cells produce native EXT2 polypeptide. If necessary, cells that have been transformed with the gene or cDNA or a vector comprising the gene or cDNA can be introduced (or reintroduced) into an individual affected with the disease. Thus, cells which, in nature, lack native EXT2 expression and activity, or have mutant EXT2 expression and activity, or have expression of a disease-associated EXT2 splicing variant, can be engineered to express EXT2 polypeptide or an active fragment of the EXT2 polypeptide (or a different variant of EXT2 polypeptide). Nucleic acid encoding the EXT2 polypeptide, or an active fragment or derivative thereof, can be introduced into an expression vector, such as a viral vector, and the vector can be introduced into appropriate cells in an animal. Other gene transfer systems, including viral and nonviral transfer systems, can be used. Alternatively, nonviral gene transfer methods, such as calcium phosphate coprecipitation, mechanical techniques (e.g., microinjection); membrane fusion-mediated transfer via liposomes; or direct DNA uptake, can also be used.

Alternatively, a nucleic acid; a nucleic acid complementary to a nucleic acid; or a portion of such a nucleic acid (e.g., an oligonucleotide as described below), can be used in "antisense" therapy, in which a nucleic acid (e.g., an oligonucleotide) which specifically hybridizes to the mRNA and/or genomic DNA of EXT2 is administered or generated in situ. The antisense nucleic acid that specifically hybridizes to the mRNA and/or DNA inhibits expression of the EXT2 polypeptide, e.g., by inhibiting translation and/or transcription. Binding of the antisense nucleic acid can be by conventional base pair complementarity, or, for example, in the case of binding to DNA duplexes, through specific interaction in the major groove of the double helix.

An antisense construct can be delivered, for example, as an expression plasmid as described above. When the plasmid is transcribed in the cell, it produces RNA which is complementary to a portion of the mRNA and/or DNA which encodes EXT2 polypeptide. Alternatively, the antisense construct can be an oligonucleotide probe which is generated ex vivo and introduced into cells; it then inhibits expression by hybridizing with the mRNA and/or genomic DNA of EXT2. The oligonucleotide probes may be modified oligonucleotides which are resistant to endogenous nucleases, e.g., exonucleases and/or endonucleases, thereby rendering them stable in vivo. Exemplary nucleic acid molecules for use as antisense oligonucleotides are phosphoramidate, phosphothioate and methylphosphonate analogs of DNA. Additionally, general approaches to constructing oligomers useful in antisense therapy are also described, for example, by van der Krol AR et al, 1988 and Stein CA and Cohen JS, 1988. With respect to antisense DNA, oligodeoxyribonucleotides derived from the translation initiation site, e.g., between the -10 and +10 regions of EXT2 sequence, are preferred.

To perform antisense therapy, oligonucleotides (mRNA, cDNA or DNA) are designed that are complementary to mRNA encoding EXT2. The antisense oligonucleotides bind to EXT2 mRNA transcripts and prevent translation. Absolute complementarity, although preferred, is not required. A sequence "complementary" to a portion of an RNA, as referred to herein, indicates that a sequence has sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid, as described in detail above. Generally, the longer the hybridizing nucleic acid the more base mismatches with an RNA it may contain and still form a stable duplex (or triplex, as the case may be). One skilled in the art can ascertain a tolerable degree of mismatch by use of standard procedures.

The oligonucleotides used in antisense therapy can be DNA, RNA, or chimeric mixtures or derivatives or modified versions thereof, single-stranded or double-stranded. The oligonucleotides can be modified at the base moiety, sugar moiety, or phosphate backbone, for example, to improve stability of the molecule, hybridization, etc. The oligonucleotides can include other appended groups such as peptides (e.g., for targeting host cell receptors in vivo), or agents facilitating transport across the cell membrane (Letsinger RL et al, 1989; Lemaitre M et al, 1987) or the blood-brain barrier (Jaeger LB and Banks WA, 2004), or hybridization-triggered cleavage agents (van der Krol AR et al, 1988) or intercalating agents. (Zon G, 1988). To this end, the oligonucleotide may be conjugated to another molecule (e.g., a peptide, hybridization triggered cross-linking agent, transport agent, hybridization-triggered cleavage agent).

The antisense molecules are delivered to cells that express EXT2 in vivo. A number of methods can be used for delivering antisense DNA or RNA to cells; e.g., antisense molecules can be injected directly into the tissue site, or modified antisense molecules, designed to target the desired cells (e.g., antisense linked to peptides or antibodies that specifically bind receptors or antigens expressed on the target cell surface) can be administered systematically. Alternatively, a recombinant DNA construct is utilized in which the antisense oligonucleotide is placed under the control of a strong promoter (e.g., pol III or pol II). The use of such a construct to transfect target cells in the patient results in the transcription of sufficient amounts of single stranded RNAs that will form complementary base pairs with the endogenous EXT2 transcripts and thereby prevent translation of the EXT2 mRNA. For example, a vector can be introduced in vivo such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art and described above. For example, a plasmid, cosmid, YAC or viral vector can be used to prepare the recombinant DNA construct that can be introduced directly into the tissue site. Alternatively, viral vectors can be used which selectively infect the desired tissue, in which case administration may be accomplished by another route (e.g., systemically).

Endogenous EXT2 expression can be also reduced by inactivating or "knocking out" EXT2 or its promoter using targeted homologous recombination (Smithies O et al, 1985; Thomas KR and Capecchi MR, 1987; Thompson S et al, 1989). For example, a mutant, non-functional EXT2 (or a completely unrelated DNA sequence) flanked by DNA homologous to the endogenous EXT2 (either the coding regions or regulatory regions of EXT2) can be used, with or without a selectable marker and/or a negative selectable marker, to transfect cells that express EXT2 in vivo. Insertion of the DNA construct, via targeted homologous recombination, results in inactivation of EXT2. The recombinant DNA constructs can be directly administered or targeted to the required site in vivo using appropriate vectors, as described above. Alternatively, expression of non-mutant EXT2 can be increased using a similar method: targeted homologous recombination can be used to insert a DNA construct comprising a non-mutant, functional EXT2 (e.g., a gene having SEQ ID NO: 1 which may optionally comprise at least one polymorphism shown in Tables 9 and 10), or a portion thereof, in place of a mutant EXT2 in the cell, as described above. Targeted homologous recombination can be used to insert a DNA construct comprising a nucleic acid that encodes an EXT2 polypeptide variant that differs from that present in the cell.

Alternatively, endogenous EXT2 expression can be reduced by targeting deoxyribonucleotide sequences complementary to the regulatory region of EXT2 (i.e., the EXT2 promoter and/or enhancers) to form triple helical structures that prevent transcription of EXT2 in target cells in the body. (Helene C, 1991; Helene C et al, 1992; Maher LJ, 1992). Likewise, the antisense constructs described herein, by antagonizing the normal biological activity of one of the EXT2 proteins, can be used in the manipulation of tissue, e.g., tissue differentiation, both in vivo and for ex vivo tissue cultures. Furthermore, the anti-sense techniques (e.g., microinjection of antisense molecules, or transfection with plasmids whose transcripts are anti-sense with regard to an EXT2 mRNA or gene sequence) can be used to investigate role of EXT2 in developmental events, as well as the normal cellular function of EXT2 in adult tissue. Such techniques can be utilized in cell culture, but can also be used in the creation of transgenic animals.

Other EXT2 therapeutic agents as described herein can also be used in the treatment or prevention of T2D. The therapeutic agents can be delivered in a composition, as describe above, or by themselves. They can be administered systemically, or can be targeted to a particular tissue. The therapeutic agents can be produced by a variety of means, including chemical synthesis; recombinant production; in vivo production, e.g. a transgenic animal (Meade H et al, 1990) and can be isolated using standard means such as those described herein.

A combination of any of the above methods of treatment (e.g., administration of non-mutant EXT2 polypeptide in conjunction with antisense therapy targeting mutant EXT2 mRNA; administration of a first splicing variant encoded by EXT2 in conjunction with antisense therapy targeting a second splicing encoded by EXT2), can also be used.

The invention will be further describe by the following non-limiting examples.

### EXPERIMENTAL SECTION

### Example 1. Genome-Wide Scanning (GWS) study

### East Finnish T2D Patients and Phenotype Characterization

The subjects were participants of the Kuopio Ischaemic Heart Disease Risk Factor Study (KIHD), which is an ongoing prospective population-based study designed to investigate risk factors for chronic diseases, including T2D and cardiovascular diseases, among middle-aged men. The study population was a random age-stratified sample of men living in Eastern Finland who were 42, 48, 54 or 60 years old at baseline examinations in 1987-1989. Repeat examinations for those who had undergone carotid ultrasound at baseline were carried out in 1991-1994 (four-year follow up) and 1998-2001 (11-year follow up). The male cohort was complemented by a random population sample of 920 women, first examined during 1998-2001, at the time of the 11-year follow up of the male cohort. In all, 854 men and 920 women participated in the 11-year follow-up. The recruitment and examination of the subjects has been described previously in detail. The University of Kuopio Research Ethics Committee approved the study. All participants gave their written informed consent.

Subjects were asked to fast for 12 hours before blood sampling, which was done between 8 and 10 a.m. They were also asked to refrain from smoking for 12 hours and from consuming alcohol for three days before blood draw. Blood glucose was measured using a glucose dehydrogenase method after precipitation of proteins by trichloroacetic acid. Diabetes was defined as fasting blood glucose concentration ≥ 6.7 mmol/l or a prior clinical diagnosis of diabetes with either dietary, oral or insulin treatment.

The cases were men and women who had T2D at the 11-year follow-up examination and at least one affected family member with T2D, who was either a parent or a sibling of the case. There were 51 such T2D cases in the KIHD 11-year follow-up database. Of these, 21 were women and 30 were men. Of the 51 diabetic cases, 28 had an oral antidiabetic medication and 17 injectable insulin treatment, while six had only dietary treatment. For each case, a diabetes-free control who had no family history of diabetes (among parents or siblings) was matched according to gender and age. For each case, a matching control was selected with the lowest fasting blood glucose level among all remaining KIHD 11-year follow-up participants. For the initial GWS, a random subset of 15 cases and 15 matched controls were selected.

### Genotyping assay

Genotyping SNP markers was performed by using the Affymetrix early access Human 100K genotyping assay. The assay consists of two arrays, Xba and Hind, which denote the restriction digestion enzymes used in these assays, and yields theoretically more than 126,000 individual genotypes. A total of 250 ng of genomic DNA in reduced EDTA TE buffer (50 ng/µl) was used for each individual assay. The DNA was digested with either XbaI or HindIII (New England Biolabs, NEB) in the mixture ofNE Buffer 2 (1 x; NEB), bovine serum albumin (1 x; NEB), and either Xba I or Hind III (0,5 U/ µl; NEB) for 2h at +37°C followed by enzyme inactivation for 20 min at +70°C. Xba I or Hind III adapters were then ligated to the samples by adding Xba or Hind II adapter (0,25µM, Affymetrix), T4 DNA ligase buffer (1 x; NEB), and T4 DNA ligase (250 U; NEB) into the digested DNA samples. Ligation reactions were allowed to proceed for 2h at +16°C followed by 20 min incubation at +70°C. Each ligated DNA sample was diluted with 75 µl of H₂0 (BioWhittaker Molecular Applications/Cambrex). Diluted DNA samples were subjected to four identical 100 µl volume polymerase chain reactions (PCR) by implementing an aliquot of 10 µl of DNA sample with Pfx Amplification Buffer (1 x; Invitrogen), PCR Enhancer (1 x; Invitrogen), MgSO₄ (1 mM; Invitrogen), dNTP (300 µM, Takara), PCR primer (1 µM, Affymetrix), and Pfx Polymerase (0,05 U/µl; Invitrogen). The PCR was allowed to proceed for 3 min at +94°C, followed by 30 cycles of 15 sec at +94°C, 30 sec at +60°C, 60 sec at +68°C, and finally for the final extension for 7 min at +68°C. The performance of the PCR was checked by standard 2% agarose gel electrophoresis in 1 x TBE buffer for 1h at 120V. PCR products were purified according to Affymetrix manual using MinElute 96 UF PCR Purification kit (Qiagen) by combining all four PCR products of an individual sample into same purification reaction. The purified PCR products were eluted with 40 µl of EB buffer (Qiagen), and the yields of the products were measured at the absorbance 260 nm. A total of 40 µg of each PCR product was then subjected to fragmentation reaction consisting of 0.2 U/µl fragmentation reagent (Affymetrix) and 1x Fragmentation Buffer. Fragmentation reaction was allowed to proceed for 35 min at +37°C followed by 15 min incubation at +95°C for enzyme inactivation. Fragmented PCR products were then checked for completeness of fragmentation by running a 4% agarose gel electrophoresis in 1 x TBE buffer (BMA Reliant precast) for 30-45 min at 120V. Fragmented PCR products were then labeled using 1 x Terminal Deoxinucleotidyl Transferase (TdT) buffer (Affymetrix), GeneChip DNA Labeling Reagent (0,214 mM; Affymetrix), and TdT (1,5 U/µl; Affymetrix) for 2h at +37°C followed by 15 min at +95°C. Labeled DNA samples were combined with hybridization buffer consisting of 0,056 M MES solution (Sigma), 5% DMSO (Sigma), 2,5 x Denhardt's solution (Sigma), 5,77 mM EDTA (Ambion), 0,115 mg/ml Herring Sperm DNA (Promega), 1 x Oligonucleotide Control reagent (Affymetrix), 11,5 µg/ml Human Cot-1 (Invitrogen), 0,0115% Tween-20 (Pierce), and 2,69 M Tetramethyl Ammonium Chloride (Sigma). DNA-hybridization mix was denatured for 10 min at +95°C, cooled on ice for 10 sec and incubated for 2 min at +48°C prior to hybridization onto GeneChip array. Hybridization was completed at +48°C for 16-18 h at 60 rpm in an Affymetrix GeneChip Hybridization Oven. Following hybridization, the arrays were stained and washed in GeneChip Fluidics Station 450 according to recommended protocol Mapping10Kv1_450. Arrays were scanned with GeneChip 2500 Scanner and the genotype calls for each of the SNP probes on the array were generated using Affymetrix Genotyping Tools (GTT) software.

### SNP selection for statistical analyses

Prior to statistical analysis SNP genotype data quality was measured based on three values: call rate (CR), minor allele frequency (MAF), and Hardy-Weinberg equilibrium (H-W). Call rate gives the proportion of samples with successful genotyping result. It does not consider if the genotypes are correct or not.

Call rate is calculated as: CR = number of samples with successful genotype call / total number of samples. Minor allele frequency (MAF) is the frequency of the allele that is less frequent in the study sample. MAF is calculated as: MAF = min(p , q), where p is frequency of the SNP allele 1 and q is frequency of the SNP allele 2; p = (number of samples with 11-genotype + 0.5*number of samples with 12-genotype) / total number of samples with successful genotype call; q = 1 - p. SNPs that are homozygous (MAF=0) are not usable in genetic analysis. Hardy-Weinberg (H-W) equilibrium is tested for controls. Test is based on standard Chi-square test of goodness of fit. Observed genotype distribution is compared to expected genotype distribution under H-W equilibrium. For two alleles this distribution is p², 2pq, and q² for genotypes AA, AB and BB, respectively, where p = f(A) and q = 1-p. If the SNP is not in H-W equilibrium it can be due to genotyping error or some unknown population dynamics (e.g. random drift, selection).

Only the SNPs that had CR > 50%, MAF > 5%, and were in H-W equilibrium (Chi-square test statistic < 6.635) were used in the statistical analysis. A total of 72,090 SNPs fulfilled the above criteria and were included in the statistical analysis.

### Statistical methods

The data set was analyzed with HPM-G program which is based on haplotype pattern mining (Toivonen et al, 2000). For phase unknown genotypic data HPM-G finds all haplotype patterns that fit the genotype configuration. The length of the haplotype patterns can vary and mutations in SNPs in each haplotype are allowed. HPM-G is more powerful than single SNP comparisons because it takes advantage of regular haplotypes. It can be used for genotype data (no need to estimate haplotypes) and does not require family data. HPM-G is very fast and can handle a large number of SNPs in a single run. SNPs are scored based on the number of times it is included in a haplotype pattern that differs between cases and controls (a threshold chi-square value can be selected by the user). Significance of the score values is evaluated based on permutation tests. The HPM-G program was run with following parameters: Chi-square threshold value (9.0), maximum haplotype pattern length (10 SNPs), maximum number of wildcards that can be included in a haplotype pattern (2 SNPs). Wildcards allow gaps in haplotypes.

### Results based on GWS

Based on the HPM-G analysis the most significant genomic region was observed on chromosome 11p11 from 43,678,152 to 44,345,353 bp from the p-term. This region had P-value < 0.0001. This region includes following genes based on the NCBI Map Viewer (genome build 34): HSD17B12, DEPC-1, LOC387763, LOC399884, LOC390110, PHACS, EXT2, and ALX4.

### Example 2: Fine-mapping of 11p11 region from 43,678,152 to 44,345,353 bp from the p-term

For the discovered region, 17 SNP markers (rs7106967 (HSD17B12 intron), rs4755736 (HSD17B12 intron), rs4755741 (HSD17B12 intron), rs1878851 (HSD17B12 intron), rs6485464 (HSD17B12 intron), rs1518820 (HSD17B12 intron), rs1518818 (DEPC-1 intron), rs2292889 (DEPC-1 UTR), rs2056248 (LOC387763 intron), rs546614, rs886196, rs2863032, rs7942915, rs1073368, rs3814767 (EXT2 unclass), rs4379834 (EXT2 intron), and rs962848 (EXT2 intron)) were genotyped from 102 subjects (51 cases and 51 controls, defined as above) that were from the same KIHD cohort as the original 30 subjects used in the GWS study. Genotypes were assessed with Applied Biosystem's SNaPshot assay using ABI Prism 3100 Genetic Analyzer (Applied Biosystems) as described below.

### Polymerase chain reaction (PCR)

The genomic DNA fragments containing the SNP markers that we genotyped (Table 2.) were amplified in four different multiplex PCR reactions (herein referred as "PCR pools 1, 2, 3 or 4"). PCR pool 1 included the amplicons for the SNPs 1, 4, 10, 15 and 19, PCR pool 2 included the amplicons for the SNPs 2, 6, 9, 13 and 18, PCR pool 3 included the amplicons 3, 11, 14 and 17, and PCR pool 4 included the amplicons 5, 8, and 12.

All multiplex PCR reactions were conducted in a 10 µl volume using using 20 ng of genomic DNA in each reaction. Compositions of the multiplex PCR reaction mixtures are presented in table 3. The PCR program for the pool 1 was: at 94°C for 7 min, 35 X (at 94°C for 45 sec, at 56°C for 30 sec, at 72°C for 2 min), and final extension at 72°C for 7 min. For the pools 2 to 4 the PCR program was: at 94°C for 7 min, 35 X (at 94°C for 45 sec, at 55°C for 30 sec, at 72°C for 2 min), and final extension at 72°C for 7 min. After amplification PCR products were stored at +4°C. The PCR amplifications were conducted with the PTC-220 DNA Engine Dyad PCR machine (MJ Research).

### Purification of the PCR products for SNaPshot reaction

All of the four PCR product pools (pools 1 to 4) were purified with SAP (Shrimp Alkaline Phosphatase, USB Corporation) and *Exo*I (Exonuclease I, New England BioLabs Inc.) treatment. This was done to avoid the participation of the unincorporated dNTPs and primers from the PCR reaction to the subsequent primer-extension reaction. More specifically 2.5 µl of SAP (1U/µl), 0.25 µl of ExoI (20 U/µl), 1.0 µl of buffer (10 X ExoI buffer, New England BioLabs Inc.) and 6.25 µl of deionized water were added to 5 µl of the PCR product. Reaction was mixed and incubated at 37°C for 1 hour, at 75°C for 15 minutes and kept at 4°C.

After the SAP/ExoI treatment 5 µl of the purified PCR products from the PCR pools 1 and 2 were combined and mixed. This same was done for the SAP/ExoI purified PCR pools 3 and 4. This procedure reduced the number of different template pools for the SNaPshot reaction (genotyping reaction) from four to only two.

**Table 2: The SNP markers (dbSNP rs ID:s are from http://www.ncbi.nih.gov/SNP/) used in fine mapping of T2D associated genomic region on chromosome 11p11 from 43,678,152 to 44,345,353 bp from the p-term. Forward and reverse primers were used to amplify genomic DNA fragments containing the SNP markers with PCR before genotyping. The sequence identification number of each primer is in parenthesis.**

| SNP id | dbSNP rs ID | Forward primer | Reverse primer | PCR product size |
|---|---|---|---|---|
| 1 | rs7106967 | ggt tac aga gtt atg ata gca g (SEQ ID NO:1) | ttt cac gaa tta gtc tta cc (SEQ ID NO:2) | 333 bp |
| 2 | rs4755736 | gtc aag ctt gca gag aat tac (SEQ ID NO:3) | agc agc aca aat gaa cta aga c (SEQ ID NO:4) | 293 bp |
| 3 | rs4755741 | tgg cta tct ctg ggc agt aag (SEQ ID NO:5) | ggg gaa tga ttt gga cag taa (SEQ ID NO:6) | 196 bp |
| 4 | rs1878851 | ctg ata ttt ttg att tgt ctc (SEQ ID NO:7) | tca atc ttt atg tgc cct tc (SEQ ID NO:8) | 423 bp |
| 5 | rs6485464 | tgt ctt ctt tca ggc aaa tg (SEQ ID NO:9) | caa cac tat gga cag aga agg (SEQ ID NO:10) | 478 bp |
| 6 | rs1518820 | atc ata gca gtg gaa aga gac (SEQ ID NO:11) | atg gtt taa aat caa ggc ag (SEQ ID NO: 12) | 448 bp |
| 8 | rs1518818 | agc agg gct ttt ggt gac aga (SEQ ID NO:13) | ctg tgg cat gca gct gat ttt (SEQ ID NO:14) | 362 bp |
| 9 | rs2292889 | gaa acg gag caa acc ttc ca (SEQ ID NO:15) | tcc cca gag gca caa gtc ca (SEQ ID NO:16) | 332 bp |
| 10 | rs2056248 | ccc cag gga aaa gca gag gag (SEQ ID NO:17) | ggg acc cat tca cag gag tag (SEQ ID NO:18) | 383 bp |
| 11 | rs546614 | cgc tgc cat aat gga aac ct (SEQ ID NO:19) | tgc ctt ttc ctt tca tct ct (SEQ ID NO:20) | 290 bp |
| 12 | rs886196 | tgt ggg att tct gta gga gat (SEQ ID NO:21) | gca gca aag aac atg aat agg t (SEQ ID NO:22) | 306 bp |
| 13 | rs2863032 | ctg gga cat gca aga aaa ag (SEQ ID NO:23) | cag aat ttc cat gaa cat aac (SEQ ID NO:24) | 475 bp |
| 14 | rs7942915 | aag ggt gtc tca gat ctg tgt (SEQ ID NO:25) | gat agg gag acc gag taa gtg (SEQ ID NO:26) | 416 bp |
| 15 | rs 1073368 | cac cca gcc gac taa ttc ttt (SEQ ID NO:27) | aag aca tgc ccc aat gaa cac (SEQ NO:28) | 175 bp |
| 17 | rs3814767 | gga tac agt tcc agt ggt gat t (SEQ ID NO:29) | ggg gat ggg aca ctc atg tt (SEQ ID NO:30) | 163 bp |
| 18 | rs4379834 | tac tgg ctg ctt ccc tta aac (SEQ ID NO:31) | gct tcc cat cat cag ata ctt (SEQ ID NO:32) | 391 bp |
| 19 | rs962848 | tgc ttt gcc atg tag gtt att (SEQ ID NO:33) | aag gag gct aaa gag aca tga (SEQ ID NO:34) | 478 bp |

**Table 3: Compositions of the multiplex PCR reactions used to amplify SNP markers used in fine mapping. Reaction volume in each pool was 10.0 µl.**

| PCR pool | Reagent | Volume for one reaction |
|---|---|---|
| 1 | 10 X buffer (QIAGEN) | 1.0 µl |
| | dNTP (10 mM) (Finnzymes) | 0.1 µl |
| | SNP 1 F+R primers (20 pmol/µl) | 1.0+1.0 µl |
| | SNP 4 F+R primers (20 pmol/µl) | 1.0+1.0 µl |
| | SNP 10 F+R primers (20 pmol/µl) | 0.3+0.3 µl |
| | SNP 15 F+R primers (20 pmol/µl) | 0.3+0.3 µl |
| | SNP 19 F+R primers (20 pmol/µl) | 0.5+0.5 µl |
| | HotStarTaq DNA Polymerase (QIAGEN) | 0.1 µl |
| | Deinonized water | 1.6 µl |
| 2 | 10 X buffer (QIAGEN) | 1.0 µl |
| | dNTP (10 mM) | 0.1 µl |
| | SNP 2 F+R primers (20 pmol/µl) | 0.5+0.5 µl |
| | SNP 6 F+R primers (20 pmol/µl) | 0.5+0.5 µl |
| | SNP 9 F+R primers (20 pmol/µl) | 0.5+0.5 µl |
| | SNP 13 F+R primers (20 pmol/µl) | 0.5+0.5 µl |
| | SNP 18 F+R primers (20 pmol/µl) | 0.5+0.5 µl |
| | HotStarTaq DNA Polymerase (QIAGEN) | 0.1 µl |
| | Deinonized water | 2.8 µl |
| 3 | 10 X buffer (QIAGEN) | 1.0 µl |
| | dNTP (10 mM) | 0.1 µl |
| | SNP 3 F+R primers (20 pmol/µl) | 0.5+0.5 µl |
| | SNP 11 F+R primers (20 pmol/µl) | 0.5+0.5 µl |
| | SNP 14 F+R primers (20 pmol/µl) | 0.5+0.5 µl |
| | SNP 17 F+R primers (20 pmol/µl) | 0.5+0.5 µl |
| | HotStarTaq DNA Polymerase (QIAGEN) | 0.1 µl |
| | Deinonized water | 3.8 µl |
| 4 | 10 X buffer (QIAGEN) | 1.0 µl |
| | dNTP (10 mM) | 0.1 µl |
| | SNP 5 F+R primers (20 pmol/µl) | 0.5+0.5 µl |
| | SNP 8 F+R primers (20 pmol/µl) | 0.5+0.5 µl |
| | SNP 12 F+R primers (20 pmol/µl) | 0.5+0.5 µl |
| | HotStarTaq DNA Polymerase (QIAGEN) | 0.1 µl |
| | Deinonized water | 5.8 µl |

### Primer extension reaction (SNaPshot reaction)

In the subsequent primer extension reaction (SNaPshot reaction) 1.5 µl of SNaPshot Multiplex Ready Reaction Mix (Applied Biosystems), 3 µl of purified pooled PCR products (combined PCR pools 1 and 2 and pools 3 and 4), 1 µl of pooled extension (SNaPshot) primers (pools contained 0.4-4 pmol of each extension primer) and 4.5 µl buffer (1 X AmpliTag Gold buffer, 2 mM MgCl2, Applied Biosystems) were mixed in a tube. The reactions were incubated at 96°C for 5 seconds and then subject to 35 cycles of 96°C for 10 sec, 50°C for 5 sec and 60°C for 30 sec in a PTC-220 DNA Engine Dyad PCR machine (MJ Research). The nucleotide sequences of the SNaPshot primers are presented in table 4.

**Table 4. Nucleotide sequences of the snapshot primers used in genotyping, variable bases present in each SNP and expected size of the SNaPshot products. The dbSNP identification numbers are from http://www.ncbi.nih.gov/SNP/ and the sequence identification number of each primer is in parenthesis.**

| SNP id | dbSNP rs ID | Nucleotide sequence of the snapshot primer (sequence ID number of the primer in the parenthesis) | Alleles in the SNP locus | Extension product size (bp) |
|---|---|---|---|---|
| **1** | rs7106967 | ttt ttt gtt agc ctg tta cca ata (SEQ ID NO:35). | G>A | 24 |
| **2** | rs4755736 | t ttt ttt ttt ctt gtc tct gtt tca gtc (SEQ ID NO:36) | G>T | 28 |
| **3** | rs4755741 | ttt ttt tgc caa caa ttt tag gga (SEQ ID NO:37) | A>G | 24 |
| **4** | rs1878851 | tt ttt ttt ttt ttt agt att ttt gac ccg tca (SEQ ID NO:38) | C>T | 32 |
| **5** | rs6485464 | ttt ttt ttt gtt aac aaa gtg tga tta c (SEQ ID NO:39) | C>T | 28 |
| **6** | rs1518820 | ttt ttt ttt ttt ttt ttt agg gaa att ctt gca ctt (SEQ ID NO:40) | G>T | 36 |
| **8** | rs1518818 | tt ttt ttt ttt ttt aag gtg gtt gtg tta ata (SEQ ID NO:41) | G>T | 32 |
| **9** | rs2292889 | t ttt ttt ttt ttt ttt ttt ttt cta acc aca gct caa aat (SEQ ID NO:42) | G>C | 40 |
| **10** | rs2056248 | tt ttt ttt ttt ttt ttt ttt ttt ttt cca cct tga tgt gca tcc (SEQ ID NO:43) | C>T | 44 |
| **11** | rs546614 | ttt ttt ttt ttt ttt ttt tgc aaa gaa aag aga tga (SEQ ID NO:44) | A>C | 36 |
| **12** | rs886196 | t ttt ttt ttt ttt ttt ttt ttt tat cac agg atc tta tca (SEQ ID NO:45) | A>G | 40 |
| **13** | rs2863032 | ttt ttt ttt ttt ttt ttt ttt ttt ttt ttt att ata ttc tag gct tgg (SEQ ID NO:46) | C>T | 48 |
| **14** | rs7942915 | tt ttt ttt ttt ttt ttt ttt ttt ttt cag tcc atg cta cct tca (SEQ ID NO:47) | C>T | 44 |
| **15** | rs1073368 | t ttt ttt ttt ttt ttt ttt ttt ttt ttt ttt ttt get gag aaa ctt att gta (SEQ ID N0:48) | A>G | 52 |
| **17** | rs3814767 | t ttt ttt ttt ttt ttt ttt ttt ttt W ttt ttt caa aat gta gca cac ace (SEQ ID N0:49) | A>G | 52 |
| **18** | rs4379834 | tt ttt ttt ttt ttt ttt ttt ttt ttt ttt ttt ttt ttt tct tcc tgt gaa gta gac (SEQ ID NO:50) | C>T | 56 |
| **19** | rs962848 | ttt ttt ttt ttt ttt ttt ttt ttt ttt ttt ttt ttt ttt ttt ctt cac cca gat tct tca (SEQ ID NO:51) | C>T | 60 |

### Post-extension treatment

After the primer extension reaction 1 unit of SAP (1U/µl) was added to the reaction mix and the reaction was incubated at 37°C for 1 hour. The enzyme was inactivated by incubating the reaction mix at 75°C for 15 minutes. Afterwards the samples were placed at 4°C. The post-extension treatment was done to prevent the unincorporated fluorescent ddNTPs obscuring the primer extension products (SNaPshot products) during electrophoresis with ABI Prism 3100 Genetic Analyzer.

### Capillary electrophoresis with ABI Prism 3100 Genetic Analyzer

Aliquots of 1 µl of pooled SNaPshot products, 9.25 µl of Hi-Di formamide (Applied Biosystems) and 0.25 µl GeneScan-120 LIZ size standard (Applied Biosystems) were combined in a 96-well 3100 optical microamp plate (Applied Biosystems). The reactions were denatured by placing them at 95°C for 5 minutes and then loaded onto an ABI Prism 3100 Genetic Analyzer (Applied Biosystems). Elelctrophoresis data was processed and the genotypes were visualized by using the GeneScan Analysis version 3.7 (Applied Biosystems).

### Statistical Methods

Allele and genotype distributions between cases and controls are tested for all SNPs. Testing is based on the standard Chi-square independence test with 1 df. Also haplotypes are tested with Chi-square test. Odds ratio (OR) and 95% confidence interval (CI) of OR were calculated for alleles and haplotypes. Haplotype patterns were created with HPM-G program.

### Results of FM

Based on the genotype distribution between cases and controls the SNPs that gave statistically significant P-values (P < 0.05) are shown in Table 5. P-value is based on Chi-square independence test with 2 df.

SNPs which gave statistically significant P-values (P < 0.05) based on the comparison of allele distribution between cases and controls are presented in Table 6. P-values are based on Chi-square independence test with 1 df. OR is the odds ratio of the T2D risk allele versus the other allele and 95% CI is the confidence interval for the odds ratio.

**Table 5. SNPs with significant (P<0.05) difference in genotype distribution between cases and controls in fine mapping.**

| dbSNP rs-id | P-value |
|---|---|
| rs546614 | 0.014 |
| rs886196 | 0.04 |
| rs2863032 | 0.014 |
| rs1073368 | 0.021 |
| rs3814767 | 0.007 |
| rs4379834 | 0.011 |
| rs962848 | 0.021 |

**Table 6. SNPs with significant (P<0.05) difference in allele distribution between cases and controls.**

| dbSNP rs-id | P-value | OR | 95% CI | T2D risk allele |
|---|---|---|---|---|
| rs546614 | 0.034 | 1.82 | 1.04<OR<3.18 | C |
| rs2863032 | 0.023 | 2.69 | 1.12<OR<6.47 | T |
| rs3814767 | 0.012 | 2.13 | 1.18<OR<3.86 | G |
| rs4379834 | 0.009 | 2.17 | 1.21<OR<3.89 | T |
| rs962848 | 0.015 | 2.04 | 1.15<OR<3.61 | T |

For example, three SNPs that are located in EXT2 introns had the following allele distribution between cases and controls: rs3814767 (SEQ ID NO:93) (59 G-alleles in controls, 43 A-alleles in controls, 76 G-alleles in cases, and 26 A-alleles in cases); rs4379834 (SEQ ID NO:94) (46 C-alleles in controls, 56 T-alleles in controls, 28 C-alleles in cases, and 74 T-alleles in cases); rs962848 (SEQ ID NO:97) (48 C-alleles in controls, 54 T-alleles in controls, 31 C-alleles in cases, and 71 T-alleles in cases).

The haplotype analysis was performed with the HPM-G software for the 17 SNPs typed in fine-mapping (Table 2.). Haplotype "GGGTG"(or nucleotides from the complementary strand), defined by the SNP markers rs1518820 (G/T) (SEQ ID NO:84), rs1518818 (G/T) (SEQ ID NO:85), rs886196 (A/G) (SEQ ID NO:89), rs2863032 (C/T) (SEQ ID NO:90) and rs3814767 (A/G) (SEQ ID NO:93), was present in 50 cases and in 33 controls. The haplotype was not present in one case and 18 controls. This corresponds to a Chi-square value of 18.69 (P-value = 0.00001) and an Odds Ratio of 27.27 (95% CI: 3.47 to 214.22).

### Example 3: Partial resequencing of EXT2 gene

The coding sequences of the EXT2 gene were partially sequenced from the 102 samples used in fine mapping in order to find sequence variants present in the EXT2 gene.

The PCR (polymerase chain reaction) amplification was conducted in a 20 µL volume. The reaction mixture contained 10 ng human genomic DNA (extracted from peripheral blood), 1 x PCR Buffer (QIAGEN), 100 µM of each of the nucleotides (dATP, dCTP, dGTP, dTTP, Finnzymes), 20 pmol of the PCR primer pairs (Table 7.) and 1 unit of the DNA-polymerase (HotStartTaq, QIAGEN). The PCR was conducted with the PTC 220 DYAD thermocycler (MJ

Research) where the program was: 94°C 7 min, 35X (94°C 45 s, annealing temperature 30 s, 72°C 2 min) 72°C 5 min and hold at 4°C. Depending on the PCR amplicon the annealing temperature varied between 51°C and 65°C. Prior the sequencing reaction, the PCR amplicons were purified with the GFX ™96 PCR Purification Kit (Amersham Pharmacia Biotech Inc, Piscataway, NJ).

**Table 7. The nucleotide sequences of the PCR primer pairs (in 5' to 3' direction) that were used to amplify the EXT2 gene target exons. Sequence identification number of each primer is in parenthesis.**

| Target exon | F-primer nucleotide sequence (SEQ ID) | R-primer nucleotide sequence (SEQ ID) | PCR product size (bp) |
|---|---|---|---|
| 12 | tgaatggaggaatggcgagg (SEQ ID:52) | gggtgacctgggcttgaacta (SEQ ID:53) | 399 |
| 11 | catgggatttacagtagtagac (SEQ ID:54) | cgcatcaatcatagaacctt (SEQ ID:55) | 606 |
| 10 | caaatcagggcagttgagttg (SEQ ID:56) | agcacctgaatgataaaatgg (SEQ ID:57) | 777 |
| 9 | atctcccctgacacagttctac (SEQ ID:58) | cgccagcttcttcacttattg (SEQ ID:59) | 693 |
| 8 | gttctcagctccttttccagt (SEQ ID:60) | caccctagaacaagaatgagat (SEQ ID:61) | 561 |
| 7 | ggcacccccatccctacaact (SEQ ID:62) | gcctctgccacaatcttgagc (SEQ ID:63) | 776 |
| 5 | tagtacactagggcctaaagag (SEQ ID:64) | ctgctctagaccagtgtactaa (SEQ ID:65) | 634 |
| 4 | cagtggaggtgaagactggta (SEQ ID:66) | catgtccagtaaagagcaatg (SEQ ID:67) | 464 |
| 3 | ccaaccagtcttcccatgcag (SEQ ID:68) | agggaaaccacataggaagcc (SEQ ID:69) | 915 |

The sequencing reactions were made by using the BigDye Terminator Cycle Sequencing v2.0 Ready Reactions with AmpliTaq DNA Polymerase, FS DNA Sequencing Kit (Applied Biosystems) and contained 4 µL RR MIX, 2 µL PCR product, 2 µL sequencing primer (2 pmol/µL) and 2 µL water. The sequencing primers are listed in table 8. Cycle sequencing was conducted with PTC 220 DYAD thermocycler (MJ Research) where the program was: 25 cycles; 10 sec at 96°C, 5 sec at 50°C and 4 min at 60°C and hold at 4°C. Dye terminator removal and sequencing reaction clean up was made using MultiScreen® -HV filtration plate (Millipore, Bedford, MA). After the purification the samples were transferred to MicroAmp® Optical 96-Well Reaction Plate (Applied Biosystems, Foster City, CA) and sequenced by using the ABI PRISM® 3100 Genetic Analyzer (Applied Biosystems, Foster City, CA) and analyzed with the Sequencing Analysis Software (Applied Biosystems) and the SeqManII program (DNASTAR).

**Table 8. The nucleotide sequences of the primers (in 5' to 3' direction) used in the resequencing of the EXT2 gene. Sequence identification number of each primer is in parenthesis. FS, forward sequencing primer; RS, reverse sequencing primer.**

| **Target of the sequencing primer** | **Nucleotide sequence of the primer (SEQ ID)** |
|---|---|
| exon 12 RS | tgctgtccttatatcttc (SEQ ID:70) |
| exon 11 RS | cgcatcaatcatagaacc (SEQ ID:71) |
| exon 10 FS | atcccattatgaccttct (SEQ ID:72) |
| exon 9 FS | gatacaagctgattctcc (SEQ ID:73) |
| exon 8 FS | gttctcagctccttttcc (SEQ ID:74) |
| exon 7 FS | gaattagcctaacctgga (SEQ ID:75) |
| exon 5 FS | aacccttgtagaaactttg (SEQ ID:76) |
| exon 4 FS | aggtgaagactggtaagga (SEQ ID:77) |
| exon 3 RS | ccctgtaactgatgtattg (SEQ ID:78) |

### Results of resequencing

In resequencing we found 5 SNPs present in EXT2 gene and three of them were associated with T2D according to statistical analyses. The results are summarized in Table 9. For example the frequency of the TT genotype and the T allele of the SNP rs4755233 (C/T) (SEQ ID NO:98) was statistically significantly more frequent in T2D patients than in healthy controls (P=0.02) indicating that the genotype TT or the allele T at this SNP locus are associated with elevated risk of T2D. Similarly the presence of the genotype TT or allele T at the SNP locus rs11037909 (C/T) (SEQ ID NO:99) is associated with increased risk of T2D. Also, in the case of SNP rs3740878 (C/T) (SEQ ID NO:100), our results show that the frequency of genotype TT or the allele T are more pronounced in T2D patients and thus are associated with increased risk of T2D.

**Table 9. The genotype and allele frequencies of the EXT2 SNPs (SNPs 1-5) found from controls and diabetic patients (T2D) by resequencing. SNP3, SNP4 and SNP5 genotype and allele frequencies deviated statistically significantly between T2D patient and control croups (denoted with an asterisk*, P≤0.02). The dbSNP rs identification numbers are presented in parenthesis, if available, according to dbSNP database (http://www.ncbi.nih.gov/SNP/).**

| | | **Controls** | | **T2D** | | | |
|---|---|---|---|---|---|---|---|
| | | **N** | **Freq.** | **N** | **Freq.** | **χ²** | **P** |
| **SNP1** | | | | | | | |
| genotypes | TT | 42 | (0.89) | 44 | (0.90) | 0.01 | 0.94 |
| | CT | 5 | (0.11) | 5 | (0.10) | | |
| | CC | 0 | (0.00) | 0 | (0.00) | | |
| | | | | | | | |
| alleles | T | 89 | (0.95) | 93 | (0.95) | 0.01 | 0.95 |
| | C | 5 | (0.05) | 5 | (0.05) | | |
| | | | | | | | |

| **SNP2 (rs12791572)** | | | | | | | |
|---|---|---|---|---|---|---|---|
| genotypes | GG | 43 | (0.92) | 46 | (0.94) | 0.20 | 0.65 |
| | CG | 4 | (0.08) | 3 | (0.06) | | |
| | CC | 0 | (0.00) | 0 | (0.00) | | |
| | | | | | | | |
| alleles | G | 90 | (0.96) | 95 | (0.97) | 0.20 | 0.66 |
| | C | 4 | (0.04) | 3 | (0.03) | | |
| | | | | | | | |

| **SNP3 (rs4755233)** | | | | | | | |
|---|---|---|---|---|---|---|---|
| genotypes | TT | 11 | (0.22) | 22 | (0.45) | 7.90 | 0.02* |
| | CT | 31 | (0.62) | 25 | (0.51) | | |
| | CC | 8 | (0.16) | 2 | (0.04) | | |
| | | | | | | | |
| alleles | T | 53 | (0.53) | 69 | (0.70) | 6.34 | 0.01* |
| | C | 47 | (0.47) | 29 | (0.30) | | |
| | | | | | | | |

| **SNP4 (rs11037909)** | | | | | | | |
|---|---|---|---|---|---|---|---|
| genotypes | TT | 13 | (0.27) | 24 | (0.49) | 9.02 | 0.01* |
| | CT | 28 | (0.57) | 24 | (0.49) | | |
| | CC | 8 | (0.16) | 1 | (0.02) | | |
| | | | | | | | |
| alleles | T | 54 | (0.55) | 72 | (0.73) | 7.20 | 0.01* |
| | C | 44 | (0.45) | 26 | (0.27) | | |
| | | | | | | | |

| **SNP5 (rs3740878)** | | | | | | | |
|---|---|---|---|---|---|---|---|
| genotypes | TT | 13 | (0.26) | 23 | (0.46) | 8.50 | 0.01* |
| | CT | 30 | (0.59) | 26 | (0.52) | | |
| | CC | 8 | (0.16) | 1 | (0.02) | | |
| | | | | | | | |
| alleles | T | 56 | (0.55) | 72 | (0.72) | 6.36 | 0.01* |
| | C | 46 | (0.45) | 28 | (0.28) | | |

| | | | | | | | |
|---|---|---|---|---|---|---|---|
| χ² = Pearson Chi-Square value | | | | | | | |

### Discussion and Conclusions

Our results indicate that genetic variation in the EXT2 gene is associated with T2D. The direct involvement of EXT2 is strongly supported by the strength of association. We first identified the association using a genome-wide set of almost 80,000 SNP markers. This was supplemented by typing additional markers in the region pinpointed by the original GWS. In addition, we typed 17 densely located markers in a larger, confirmatory data set and in resequencing we found 3 additional SNP markers associated with T2D in fime mapping sample set (Table 10.). Although we have not identified a functional mutation in the EXT2 gene, we have identified a haplotype that extends over the entire EXT2 gene. This haplotype is present in 98% of the T2D patients, compared with 65% in the control group with more than 27-fold T2D probability for the carriers of this haplotype.

In summary, we have presented association analyses (single marker and haplotype analyses) that support the notion that the EXT2 gene confers greatly increased probability of T2D. We propose that this gene is involved in the pathogenesis of T2D. EXT2 is expressed in cell types important in T2D. Modification of EXT2 activity in target cells in general or specifically one or more isoforms, by a small molecule drug or other pharmacological agent might decrease the risk of T2D and its complications in general, and especially in those who are predisposed to T2D through variation in the EXT2 gene.

**Table 10. Summary of all genotyped SNP markers and their association to type 2 diabetes. P-value for SNPs with significant (P<0.05) difference in genotype distribution between cases and controls is given. The dbSNP identification numbers are from http://www.ncbi.nih.gov/SNP/. FM, fine mapping study; ns, not significant.**

| **Study** | **dbSNP rs_ID** | **SEQ ID** | **P-value** |
|---|---|---|---|
| FM | rs7106967 | SEQ ID: 79 | ns |
| FM | rs4755736 | SEQ ID: 80 | ns |
| FM | rs4755741 | SEQ ID: 81 | ns |
| FM | rs1878851 | SEQ ID: 82 | ns |
| FM | rs6485464 | SEQ ID: 83 | ns |
| FM | rs1518820 | SEQ ID: 84 | ns |
| FM | rs1518818 | SEQ ID: 85 | ns |
| FM | rs2292889 | SEQ ID: 86 | ns |
| FM | rs2056248 | SEQ ID: 87 | ns |
| FM | rs546614 | SEQ ID: 88 | 0.014 |
| FM | rs886196 | SEQ ID: 89 | 0.04 |
| FM | rs2863032 | SEQ ID: 90 | 0.014 |
| FM | rs7942915 | SEQ ID: 91 | ns |
| FM | rs1073368 | SEQ ID: 92 | 0.021 |
| FM | rs3814767 | SEQ ID: 93 | 0.007 |
| FM | rs4379834 | SEQ ID: 94 | 0.011 |
| Sequencing | IVS7+126T>C | SEQ ID: 95 | ns |
| Sequencing | rs12791572 | SEQ ID: 96 | ns |
| FM | rs962848 | SEQ ID: 97 | 0.021 |
| Sequencing | rs4755233 | SEQ ID: 98 | 0.02 |
| Sequencing | rs11037909 | SEQ ID: 99 | 0.01 |
| Sequencing | rs3740878 | SEQ ID: 100 | 0.01 |

### References

Altshuler D, Hirschhom JN, Klannemark M, Lindgren CM, Vohl MC, et al. 2000. The common PPARgamma Pro12Ala polymorphism is associated with decreased risk of type 2 diabetes. Nat. Genet. 26:76-80.
American Diabetes Association. 2003. Report of the Expert Committee on the Diagnosis and Classification of Diabetes Mellitus. Diabetes Care 26:S5-S20.
Barnett AH, Eff C, Leslie RD, Pyke DA. 1981. Diabetes in identical twins. A study of 200 pairs. Diabetologia 20:87-93.
Bell GI, Horita S, Karam JH. 1984. A polymorphic locus near the human in-sulin gene is associated with insulin-dependent diabetes mellitus. Diabetes 33:176-83.
Bell GI, Xiang K, Newman MV, Wu S, Wright LG, Fajans SS, Spielman RS, Cox NJ. 1991. Gene for non-insulin-dependent diabetes mellitus (maturity-onset diabetes of the young subtype) is linked to DNA polymorphism on human chromosome 20q. Proc Natl Acad Sci. 88:1484-1488.
Bellaiche Y, The I, Perrimon N. 1998. Tout-velu is a Drosophila homologue of the putative tumour suppressor EXT-1 and is needed for Hh diffusion. Nature 394:85-8.
Bennett ST, Todd JA. 1996. Human type 1 diabetes and the insulin gene: principles of mapping polygenes. Annu Rev Genet. 30:343-70.
Bierer B, Coligan JE, Margulies DH, Shevach EM, Strober W. 2002. Current Protocols in Immunology. NY:John Wiley & Sons.
Byrne MM, Sturis J, Menzel S, Yamagata K, Fajans SS, Dronsfield MJ, Bain SC, Hattersley AT, Velho G, Froguel P, Bell GI, Polonsky KS. 1996. Altered insulin secretory response to glucose in diabetic and nondiabetic subjects with mutations in the diabetes susceptibility gene MODY3 on chromosome 12. Diabetes 45:1503-1510.
Carnevale V, Romagnoli E, D'Erasmo E. 2004. Skeletal involvement in patients with diabetes mellitus. Diabetes Metab Res Rev. 20:196-204.
Chiu KC, Province MA, Dowse GK, Zimmet PZ, Wagner G, et al. 1992. A genetic marker at the glucokinase gene locus for type 2 (non-insulin-dependent) diabetes mellitus in Mauritian Creoles. Diabetologia 35:632-38.
Clement K, Pueyo ME, Vaxillaire M, Rakotoambinina B, Thuillier F, Passa P, Froguel P, Roberts J, Velho G. 1996. Assessment of insulin sensitivity in glucokinase-deficient subjects. Diabetologia 39:82-90.
Cole SP, Campling BG, Atlaw T, Kozbor D, Roder JC. 1984. Human monoclonal antibodies. Mol Cell Biochem. 62:109-20.
Concannon P, Gogolin-Ewens KJ, Hinds DA, Wapelhorst B, Morrison VA, et al. 1998. A second-generation screen of the human genome for susceptibility to insulin-dependent diabetes mellitus. Nat Genet. 19:292-96.
Cox NJ, Frigge M, Nicolae DL, Con-cannon P, Hanis CL, et al. 1999. Loci on chromosomes 2 (NIDDM1) and 15 interact to increase susceptibility to diabetes in Mexican Americans. Nat Genet. 21:213-15.
Cox NJ, Wapelhorst B, Morrison VA, Johnson L, Pinchuk L, et al. 2001. Seven regions of the genome show evidence of linkage to type 1 diabetes in a consensus analysis of 767 multiplex families. Am J Hum Genet. 69:820-30.
Cudworth AG, Woodrow JC. 1974. Letter: HLA antigens and diabetes mellitus. Lancet 2:1153.
Davies JL, Kawaguchi Y, Bennett ST, Copeman JB, Cordell HJ, et al. 1994. A genome-wide search for human type 1 diabetes susceptibility genes. Nature 371:130-36.
Deeb SS, Fajas L, Nemoto M, Pihla-jamaki J, Mykkanen L, et al. 1998. A Pro12Ala substitution in PPARgamma2 associated with decreased receptor activity, lower body mass index and improved insulin sensitivity. Nat Genet. 20:284-87.
Duncan G, McCormick C, Tufaro F. 2001. The link between heparan sulfate and hereditary bone disease: finding a function for the EXT family of putative tumor suppressor proteins. J Clin Invest. 108:511-6.
Espallargues M, Sampietro-Colom L, Estrada MD, Sola M, del Rio L, Setoain J, Granados A. 2001. Identifying bone-mass-related risk factors for fracture to guide bone densitometry measurements: a systematic review of the literature. Osteoporos Int. 12:811-22.
Field LL. 2002. Genetic linkage and association studies of type I diabetes: challenges and rewards. Diabetoloiga 45:21-35.
Fields S, Song O. 1989. A novel genetic system to detect protein-protein interactions. Nature 340:245-6.
Florez JC, Hirschhorn J, Altshuler D. 2003. The inherited basis of diabetes mellitus: implications for the genetic analysis of complex traits. Annu Rev Genomics Hum Genet. 4:257-91.
Froguel P, Vaxillaire M, Sun F, Velho G, Zouali H, Butel MO, Lesage S, Vionnet N, Clement K, Fougerousse F, et al. 1992. Close linkage of glucokinase locus on chromosome 7p to early-onset non-insulin-dependent diabetes mellitus. Nature 356:162-164.
Gloyn AL, Hashim Y, Ashcroft SJ, Ash-field R, Wiltshire S, Turner RC. 2001. Association studies of variants in promoter and coding regions of beta-cell ATP-sensitive K-channel genes SUR1 and Kir6.2 with Type 2 diabetes mellitus (UKPDS 53). Diabetes Med. 18:206-12. Gloyn AL, Weedon MN, Owen KR, Turner MJ, Knight BA, et al. 2003. Large-scale association studies of variants in genes encoding the pancreatic beta-cell KATP channel subunits Kir6.2 (KCNJ11) and SUR1 (ABCC8) confirm that the KCNJ11 E23K variant is associated with type 2 diabetes. Diabetes 52:568-72.
Gough SC, Saker PJ, Pritchard LE, Mer-riman TR, Merriman ME, et al. 1995. Mutation of the glucagon receptor gene and diabetes mellitus in the UK: association or founder effect? Hum Mol Genet. 4:1609-12.
Griffiths AD, Malmqvist M, Marks JD, Bye JM, Embleton MJ, McCafferty J, Baier M, Holliger KP, Gorick BD, Hughes-Jones NC, et al. 1993. Human anti-self antibodies with high specificity from phage display libraries. EMBO J. 12:725-34.
Gross DJ, Weiss L, Reibstein I, van den Brand J, Okamoto H, Clark A, Slavin S. 1998. Amelioration of diabetes in nonobese diabetic mice with advanced disease by linomide-induced immunoregulation combined with Reg protein treatment. Endocrinology 139:2369-2374.
Hager J, Hansen L, Vaisse C, Vionnet N, Philippi A, et al. 1995. A missense mutation in the glucagon receptor gene is associated with non-insulin-dependent diabetes mellitus. Nat Genet. 9:299-304.
Han C, Belenkaya TY, Khodoun M, Tauchi M, Lin X, Lin X. 2004. Distinct and collaborative roles of Drosophila EXT family proteins in morphogen signalling and gradient formation. Development 131:1563-75.
Hani EH, Boutin P, Durand E, Inoue H, Permutt MA, et al. 1998. Missense mutations in the pancreatic islet beta cell inwardly rectifying K+ channel gene (KIR6.2/BIR): a meta-analysis suggests a role in the polygenic basis of Type II diabetes mellitus in Caucasians. Diabetologia 41:1511-15.
Hani EH, Clement K, Velho G, Vionnet N, Hager J, et al. 1997. Genetic studies of the sulfonylurea receptor gene locus in NIDDM and in morbid obesity among French Caucasians. Diabetes 46:688-94.
Hanis CL, Boerwinkle E, Chakraborty R, Ellsworth DL, Concannon P, et al. 1996. A genome-wide search for human non-insulin-dependent (type 2) diabetes genes reveals a major susceptibility locus on chromosome 2. Nat Genet 13:161-66.
Hansen T, Echwald SM, Hansen L, Moller AM, Almind K, et al. 1998. Decreased tolbutamide-stimulated insulin secretion in healthy subjects with sequence variants in the high-affinity sulfonylurea receptor gene. Diabetes 47: 598-605.
Hara K, Okada T, Tobe K, Yasuda K, Mori Y, et al. 2000. The Pro12Ala poly-morphism in PPAR gamma2 may confer resistance to type 2 diabetes. Biochem Biophys Res Commun. 271:212-16.
Hashimoto L, Habita C, Beressi JP, Delepine M, Besse C, et al. 1994. Ge-netic mapping of a susceptibility locus for insulin-dependent diabetes mellitus on chromosome 11q. Nature 371:161-64.
Hay BN, Sorge JA, Shopes B. 1992. Bacteriophage cloning and Escherichia coli expression of a human IgM Fab. Hum Antibodies Hybridomas 3:81-5.
Hayashi N, Kipriyanov S, Fuchs P, Welschof M, Dorsam H, Little M. 1995. A single expression system for the display, purification and conjugation of single-chain antibodies. Gene 160:129-30.
Helene C, Thuong NT, Harel-Bellan A. 1992. Control of gene expression by triple helix-forming oligonucleotides. The antigene strategy. Ann N Y Acad Sci. 660:27-36.
Helene C. 1991. The anti-gene strategy: control of gene expression by triplex-forming-oligonucleotides. Anticancer Drug Des. 6:569-84.
Hering S, Karawajew L, Pasternak G. 1988. Raji-K562 hybrids and their use for trioma production. Biomed Biochim Acta 47:211-6.
Herman WH, Fajans SS, Oritz FJ, Smith MJ, Sturis J, Bell GI, Polonsky KS, Halter JB. 1994. Abnormal insulin secretion, not insulin resistance, is the genetic or primary defect of MODY in the RW pedigree. Diabetes 43:40-46.
Hitman GA, Tarn AC, Winter RM, Drummond V, Williams LG, et al. 1985. Type 1 (insulin-dependent) diabetes and a highly variable locus close to the in-sulin gene on chromosome 11. Diabetologia 28:218-22.
Horikawa Y, Oda N, Cox NJ, Li X, Orho-Melander M, et al. 2000. Genetic variation in the gene encoding calpain-10 is associated with type 2 diabetes mellitus. Nat Genet. 26:163-75. Huse WD, Sastry L, Iverson SA, Kang AS, Alting-Mees M, Burton DR, Benkovic SJ, Lerner RA. 1989. Generation of a large combinatorial library of the immunoglobulin repertoire in phage lambda. Science 246:1275-81.
Ingham PW, McMahon AP. 2001. Hedgehog signaling in animal development: paradigms and principles. Genes Dev. 15:3059-87.
Inoue H, Ferrer J, Welling CM, Elbein SC, Hoffman M, et al. 1996. Sequence variants in the sulfonylurea receptor (SUR) gene are associated with NIDDM in Caucasians. Diabetes 45:825-31.
Jaeger LB, Banks WA. 2004. Antisense therapeutics and the treatment of CNS disease. Front Biosci. 9:1720-7.
Kadowaki T, Kadowaki H, Mori Y, Tobe K, Sakuta R, Suzuki Y, Tanabe Y, Sakura H, Awata T, Goto Y, et al. 1994. A subtype of diabetes mellitus associated with a mutation of mitochondrial DNA. N Engl J Med 330:962-968.
Kahn CR, Flier JS, Bar RS, Archer JA, Gorden P, Martin MM, Roth J. 1976. The syndromes of insulin resistance and acanthosis nigricans. N Engl J Med 294:739-745.
Kawahira H, Ma NH, Tzanakakis ES, McMahon AP, Chuang PT, Hebrok M. 2003. Combined activities of hedgehog signaling inhibitors regulate pancreas development. Development 130:4871-9.
Kim SK, Hebrok M. 2001. Intercellular signals regulating pancreas development and function. Genes Dev. 15:111-27.
Kobayashi S, Akiyama T, Nata K, Abe M, Tajima M, Shervani NJ, Unno M, Matsuno S, Sasaki H, Takasawa S, Okamoto H. 2000. Identification of a receptor for reg (regenerating gene) protein, a pancreatic beta-cell regeneration factor. J Biol Chem. 275:10723-6.
Kohler G, Milstein C. 1975. Continuous cultures of fused cells secreting antibody of predefined specificity. Nature 256:495-497.
Kozbar D, Lagarde AE, Roder JC. 1982. Human hybridomas constructed with antigen-specific Epstein-Barr virus-transformed cell lines. Proc Natl Acad Sci U S A. 79(21):6651-5
Kwok P-Y. 2001. Methods for genotyping single nucleotide polymorphisms. Ann Rev Genomics Hum Genet. 2:235-258.
Lam KS. 1997. Application of combinatorial library methods in cancer research and drug discovery. Anticancer Drug Des. 12:145-67.
Leidig-Bruckner G, Ziegler R. 2001. Diabetes mellitus a risk for osteoporosis? Exp Clin Endocrinol Diabetes. 109 Suppl 2:S493-514.
Lemaitre M, Bayard B, Lebleu B. 1987. Specific antiviral activity of a poly(L-lysine)-conjugated oligodeoxyribonucleotide sequence complementary to vesicular stomatitis virus N protein mRNA initiation site. Proc Natl Acad Sci U S A. 84:648-52.
Letsinger RL, Zhang GR, Sun DK, Ikeuchi T, Sarin PS. 1989. Cholesteryl-conjugated oligonucleotides: synthesis, properties, and activity as inhibitors of replication of human immunodeficiency virus in cell culture. Proc Natl Acad Sci U S A. 86:6553-6.
Li SR, Baroni MG, Oelbaum RS, Stock J, Galton DJ. 1988. Association of genetic variant of the glucose transporter with non-insulin-dependent diabetes mellitus. Lancet 2:368-70. Maher LJ 3rd. 1992. DNA triple-helix formation: an approach to artificial gene repressors? Bioessays 14:807-15.
Marron MP, Raffel LJ, Garchon H-J, Jacob CO, Serrano Rios M, et al. 1997. Insulin-dependent diabetes melli-tus (IDDM) is associated with CTLA4 polymorphisms in multiple ethnic groups. Hum Mol Genet. 6:1275-82.
McCarthy MI, Hitman GA, Hitchins M, Riikonen A, Stengard J, et al. 1994. Glucokinase gene polymorphisms: a genetic marker for glucose intolerance in a cohort of elderly Finnish men. Diabetes Med. 11:198-204.
McConnell HM, Owicki JC, Parce JW, Miller DL, Baxter GT, Wada HG, Pitchford S. 1992. The cytosensor microphysiometer: biological applications of silicon technology. Science 257:1906-12.
Meade H, Gates L, Lacy E, Lonberg N. 1990. Bovine alpha S1-casein gene sequences direct high level expression of active human urokinase in mouse milk. Biotechnology (N Y). 8:443-6.
Mein CA, Esposito L, Dunn MG, Johnson GC, Timms AE, et al. 1998. A search for type 1 diabetes susceptibility genes in families from the United Kingdom. Nat Genet. 19:297-300.
Mori H, Ikegami H, Kawaguchi Y, Seino S, Yokoi N, et al. 2001. The Pro12 → Ala substitution in PPAR-gamma is associated with resistance to development of diabetes in the general population: possible involvement in impairment of insulin secretion in individuals with type 2 diabetes. Diabetes 50:891-94.
Nerup JP, Platz P, Andersen OO, Christy M, Lyngsoe J, et al. 1974. HLA antigens and diabetes mellitus. Lancet 2:864-66.
Nielsen PE, Egholm M, Berg RH, Buchardt O. 1991. Sequence-selective recognition of DNA by strand displacement with a thymine-substituted polyamide. Science 254:1497-500.
Nisticò L, Buzzetti R, Pritchard LE, Van der Auwera B, Giovannini C, et al. 1996. The CTLA-4 gene region of chromosome 2q33 is linked to, and associated with, type 1 diabetes. Hum Mol Genet. 5:1075-80.
Perrimon N, Bernfield M. 2000. Specificities of heparan sulphate proteoglycans in developmental processes. Nature 404:725-8.
Platz P, Jakobsen BK, Morling N, Ryder LP, Svejgaard A, et al. 1981. HLA-D and -DR antigens in genetic analysis of insulin dependent diabetes mellitus. Diabetologia 21:108-15.
Pontiroli AE, Capra F, Veglia F, Ferrari M, Xiang KS, et al. 1996. Genetic contribution of polymorphism of the GLUT1 and GLUT4 genes to the susceptibility to type 2 (non-insulin-dependent) diabetes mellitus in different populations. Acta Diabetologica 33:193-97.
Poulsen P, Kyvik KO, Vaag A, Beck-Nielsen H. 1999. Heritability of type II (non-insulin-dependent) diabetes mellitus and abnormal glucose tolerance-a population-based twin study. Diabetologia 42:139-45.
Reardon W, Ross RJM, Sweeney MG, Luxon LM, Pembrey ME, Harding AE, Trembath RC. 1992. Diabetes mellitus associated with a pathogenic point mutation in mitochondrial DNA. Lancet 340:1376-1379.
Redondo MJ, Fain PR, Eisenbarth GS. 2001. Genetics of type 1A diabetes. Rec Prog Horm Res. 56:69-89.
Rotter JI, Anderson CE, Rubin R, Congleton JE, Terasaki PI, Rimoin DL. 1983. HLA genotypic study of insulin-dependent diabetes the excess of DR3/DR4 heterozygotes allows rejec-tion of the recessive hypothesis. Diabetes 32:169-74.
Sheehy MJ, Scharf SJ, Rowe JR, Neme de Gimenez MH, Meske LM, et al. 1989. A diabetes-susceptible HLA haplotype is best defined by a combination of HLA-DR and -DQ alleles. J Clin Invest. 83:830-35.
Singal DP, Blajchman MA. 1973. His-tocompatibility (HL-A) antigens, lym-phocytotoxic antibodies and tissue anti-bodies in patients with diabetes mellitus. Diabetes 22:429-32. Smith LL, Burnet SP, McNeil JD. 2003. Musculoskeletal manifestations of diabetes mellitus. Br J Sports Med. 37:30-5.
Smithies O, Gregg RG, Boggs SS, Koralewski MA, Kucherlapati RS. 1985. Insertion of DNA sequences into the human chromosomal beta-globin locus by homologous recombination. Nature 317:230-4.
Stein CA, Cohen JS. 1988. Oligodeoxynucleotides as inhibitors of gene expression: a review. Cancer Res. 48:2659-68.
Syvänen A-C. 2001. Accessing genetic variation: Genotyping single nucleotide polymorphisms. Nature Reviews Genetics. 2:930-942.
Takekawa K, Ikegami H, Fukuda M, Ueda H, Kawaguchi Y, et al. 1994. Early-onset type 2 (non-insulin-dependent) diabetes mellitus is associated with glucokinase locus, but not with adenosine deaminase locus, in the Japanese population. Diabetes Res Clin Pract. 23:141-46.
Tao T, Tanizawa Y, Matsutani A, Mat-subara A, Kaneko T, Kaku K. 1995.HepG2/erythrocyte glucose transporter (GLUT1) gene in NIDDM: a population association study and molecular scan-ning in Japanese subjects. Diabetologia 38:942-47.
Taylor SI. 1992. Lilly Lecture: molecular mechanisms of insulin resistance: lessons from patients with mutations in the insulin-receptor gene. Diabetes 41:1473-1490.
Terazono K, Yamamoto H, Takasawa S, Shiga K, Yonemura Y, Tochino Y, Okamoto H. 1988. A novel gene activated in regenerating islets. J Biol Chem. 263:2111-2114.
Thomas KR, Capecchi MR. 1987. Site-directed mutagenesis by gene targeting in mouse embryo-derived stem cells. Cell 51:503-12.
Thomas MK, Rastalsky N, Lee JH, Habener JF. 2000. Hedgehog signaling regulation of insulin production by pancreatic beta-cells. Diabetes 49:2039-47.
Thompson S, Clarke AR, Pow AM, Hooper ML, Melton DW. 1989. Germ line transmission and expression of a corrected HPRT gene produced by gene targeting in embryonic stem cells. Cell 56:313-21.
Todd JA, Bell JI, McDevitt HO. 1987. HLA-DQβ gene contributes to suscepti-bility and resistance to insulin-dependent diabetes mellitus. Nature 329:599-604.
Todd JA, Mijovic C, Fletcher J, Jenk-ins D, Bradwell AR, Barnett AH. 1989. Identification of susceptibility loci for insulin-dependent diabetes mellitus by trans-racial gene mapping. Nature 338:587-89.
Toivonen HT, Onkamo P, Vasko K, Ollikainen V, Sevon P, Mannila H, Herr M, Kere J. 2000. Data mining applied to linkage disequilibrium mapping. Am J Hum Genet 67:133-45. Vaessen N, Heutink P, Houwing-Duistermaat JJ, Snijders PJ, Rademaker T, Testers L, Batstra MR, Sandkuijl LA, van Duijn CM, Oostra BA. 2002. A genome-wide search for linkage-disequilibrium with type 1 diabetes in a recent genetically isolated population from the Netherlands. Diabetes 51:856-9.
Van den Ouwenland JMW, Lemkes HHPJ, Ruitenbeek W, Sandkuijl LA, de Vijlder MF, Struyvenberg PAA, van de Kamp, Maassen JA. 1992. Mutation in mitochondrial tRNA (Leu(URR) gene in a large pedigree with maternally transmitted type II diabetes mellitus and deafness. Nature Genet. 1:368-371.
van der Krol AR, Mol JN, Stuitje AR. 1988. Modulation of eukaryotic gene expression by complementary RNA or DNA sequences. Biotechniques 6:958-76.
Vaxillaire M, Boccio V, Philippi A, Vigouroux C, Terwilliger J, Passa P, Beckman JS, Velho G, Lathrop GM, Froguel P. 1995. A gene for maturity onset diabetes of the young (MODY) maps to chromosome 12q. Nature Genet. 9:418-423.
Vionnet N, Stoffel M, Takeda J, Yasuda K, Bell GI, Zouali H, Lesage S, Velho G, Iris F, Passa P, et al. 1992. Nonsense mutation in the glucokinase gene causes early-onset non-insulin-dependent diabetes mellitus. Nature 356:721-722.
Watanabe T, Yonekura H, Terazono K, Yamamoto H, Okamoto H. 1990. Complete nucleotide sequence of human reg gene and its expression in normal and tumoral tissues: the reg protein, pancreatic stone protein, and pancreatic thread protein are one and the same product of the gene. J Biol Chem. 265:7432-7439.
Watanabe T, Yonemura Y, Yonekura H, Suzuki Y, Miyashita H, Sugiyama K, Moriizumi S, Unno M, Tanaka O, Kondo H, Bone AJ, Takasawa S, Okamoto H. 1994. Pancreatic beta-cell replication and amelioration of surgical diabetes by Reg protein. Proc Natl Acad Sci U S A. 91:3589-3592.
World Health Organization and the International Diabetes Federation. 2004. Diabetes Action Now booklet. http://www.who.int/diabetes/actionnow/booklet/en
World Health Organization. 2004. Diabetes Mellitus fact sheet. http://www.who.int/mediacentre/factsheets/fs138/en
World Health Organization. 2004. Obesity and overweight Fact Sheet. http://www.who.int/hpr/NPH/docs/gs obesity.pdf
Wuyts W, Van Hul W, De Boulle K, Hendrickx J, Bakker E, Vanhoenacker F, Mollica F, Ludecke HJ, Sayli BS, Pazzaglia UE, Mortier G, Hamel B, Conrad EU, Matsushita M, Raskind WH, Willems PJ. 1998. Mutations in the EXT1 and EXT2 genes in hereditary multiple exostoses. Am J Hum Genet. 62:346-54.
Yamagata K, Oda N, Kaisaki PJ, Menzel S, Furuta H, Vaxillaire M, Southam L, Cox RD, Lathrop GM, Boriraj VV, Chen X, Cox NJ, Oda Y, Yano H, Le Beau MM, Yamada S, Nishigori H, Takeda J, Fajans SS, Hattersley AT, Iwasaki N, Hansen T, Pedersen O, Polonsky KS, Bell GI, et al..1996. Mutations in the hepatocyte nuclear factor-1α gene in maturity-onset diabetes of the young (MODY 3). Nature 384:455-458.
Yamagata K, Furuta H, Oda N, Kaisaki PJ, Menzel S, Cox NJ, Fajans SS, Signorini S, Stoffel M, Bell GI. 1996. Mutations in the hepatocyte factor-4α gene in maturity-onset diabetes of the young (MODY 1). Nature 384:458-460.
Yamaoka T, Itakura M. 1999. Development of pancreatic islets. Int J Mol Med. 3:247-61. Zhang YW, Ding LS, Lai MD. 2003. Reg gene family and human diseases. World J Gastroenterol. 9:2635-41.
Zon G. 1988. Oligonucleotide analogues as potential chemotherapeutic agents. Pharm Res. 5:539-49.

## Claims

1. A method for diagnosing a predisposition for type 2 diabetes in a human subject comprising:
a) providing a biological sample taken from the subject,
b) assessing one or more SNP markers in said sample, wherein said SNP markers are associated to EXT2 gene, and are located between position 43.678,152 and 44,345,353, on chromosome 11p11,
c) comparing SNP marker data obtained in step b) to SNP marker data from healthy and diseased people to diagnose a predisposition for type 2 diabetes.

2. The method according to claim 1, wherein SNP markers associated to EXT2 gene are selected from the group consisting of rs546614, rs886196, rs2863032, rs1073368, rs3814767, rs4379834, rs962848, rs4755233, rs11037909, and rs3740878.

3. The method according to claim 1 or 2, further comprising step d) combining personal and clinical information with the SNP marker data to make a diagnosis of a predisposition for type 2 diabetes.

4. The method according to claim 3, wherein the personal and clinical information concerns age, gender, obesity, the family history of obesity and diabetes, waist-to-hip circumference ratio (cm/cm), and the medical history concerning diabetes of the subject.

5. The method according to claim 3 or 4 further comprising determining blood, serum or plasma fibrinogen, ferritin, transferring receptor, C-reactive protein and insulin concentration from the subject.

6. The method according to any one of the claims 1 to 5, wherein:
(i) oligonucleotide primers selected from the group consisting of SEQ ID NO:19, SEQ ID NO:20, and SEQ ID NO:44 are used to assess rs546614,
(ii) oligonucleotide primers selected from the group consisting of SEQ ID NO:21, SEQ ID NO:22, and SEQ ID NO:45 are used to assess rs886196,
(iii) oligonucleotide primers selected from the group consisting of SEQ ID NO:23, SEQ ID NO:24, and SEQ ID NO:46 are used to assess rs2863032,
(iv) oligonucleotide primers selected from the group consisting of SEQ ID NO:27, SEQ ID NO:28, and SEQ ID NO:48 are used to assess rs1073368,
(v)oligonucleotide primers selected from the group consisting of SEQ ID NO:29, SEQ ID NO:30, and SEQ ID NO:49 are used to assess rs3814767,
(vi) oligonucleotide primers selected from the group consisting of SEQ ID NO:31, SEQ ID NO:32, and SEQ ID NO:50 are used to assess rs4379834,
(vii) oligonucleotide primers selected from the group consisting of SEQ ID NO:33, SEQ ID NO:34, and SEQ ID NO:51 are used to assess rs962848,
(viii) oligonucleotide primers selected from the group consisting of SEQ ID NO:60, SEQ ID NO:61, and SEQ ID NO:74 are used to assess rs4755233,
(ix) oligonucleotide primers selected from the group consisting of SEQ ID NO:54, SEQ ID NO:55, and SEQ ID NO:71 are used to assess rs11037909, and
(x) oligonucleotide primers selected from the group consisting of SEQ ID NO:52, SEQ ID NO:53, and SEQ ID NO:70 are used to assess rs3740878.

## Patentansprüche

1. Verfahren zur Diagnose einer Veranlagung zu Typ-2-Diabetes bei einem menschlichen Patienten, umfassend:
a) Bereitstellen einer dem Patienten entnommenen biologischen Probe,
b) Beurteilen eines oder mehrerer SNP-Marker in der Probe, wobei die SNP-Marker mit dem EXT2-Gen assoziiert sind und zwischen Position 43.678.152 und 44.345.353 auf Chromosom 11p11 liegen,
c) Vergleichen der in Schritt b) erhaltenen SNP-Marker-Daten mit SNP-Marker-Daten von gesunden und erkrankten Personen, um eine Veranlagung zu Typ-2-Diabetes zu diagnostizieren.

2. Verfahren nach Anspruch 1, wobei mit dem EXT2-Gen assoziierte SNP-Marker aus der aus rs546614, rs886196, rs2863032, rs1073368, rs3814767, rs4379834, rs962848, rs4755233, rs11037909 und rs3740878 bestehenden Gruppe ausgewählt sind.

3. Verfahren nach Anspruch 1 oder 2, ferner umfassend Schritt d), wobei persönliche und klinische Informationen mit den SNP-Marker-Daten kombiniert werden, um eine Diagnose einer Veranlagung zu Typ-2-Diabetes zu stellen.

4. Verfahren nach Anspruch 3, wobei die persönlichen und klinischen Informationen Alter, Geschlecht, Fettleibigkeit, die Familiengeschichte hinsichtlich Fettleibigkeit und Diabetes, Verhältnis von Taillen- zu Hüftumfang (cm/cm) und die Diabetes betreffende medizinische Geschichte des Patienten betreffen.

5. Verfahren nach Anspruch 3 oder 4, ferner umfassend das Bestimmen von Blut-, Serum- oder Plasma-Fibrinogen, Ferritin, Transferrin-Rezeptor, C-reaktivem Protein und Insulinkonzentration von dem Patienten.

6. Verfahren nach einem der Ansprüche 1 bis 5, wobei:
(i) aus der aus SEQ ID NO: 19, SEQ ID NO: 20 und SEQ ID NO: 44 bestehenden Gruppe ausgewählte Oligonukleotid-Primer zur Beurteilung von rs546614 eingesetzt werden,
(ii) aus der aus SEQ ID NO: 21, SEQ ID NO: 22 und SEQ ID NO: 45 bestehenden Gruppe ausgewählte Oligonukleotid-Primer zur Beurteilung von rs886196 eingesetzt werden,
(iii) aus der aus SEQ ID NO: 23, SEQ ID NO: 24 und SEQ ID NO: 46 bestehenden Gruppe ausgewählte Oligonukleotid-Primer zur Beurteilung von rs2863032 eingesetzt werden,
(iv) aus der aus SEQ ID NO: 27, SEQ ID NO: 28 und SEQ ID NO: 48 bestehenden Gruppe ausgewählte Oligonukleotid-Primer zur Beurteilung von rs1073368 eingesetzt werden,
(v) aus der aus SEQ ID NO: 29, SEQ ID NO: 30 und SEQ ID NO: 49 bestehenden Gruppe ausgewählte Oligonukleotid-Primer zur Beurteilung von rs3814767 eingesetzt werden,
(vi) aus der aus SEQ ID NO: 31, SEQ ID NO: 32 und SEQ ID NO: 50 bestehenden Gruppe ausgewählte Oligonukleotid-Primer zur Beurteilung von rs4379834 eingesetzt werden,
(vii) aus der aus SEQ ID NO: 33, SEQ ID NO: 34 und SEQ ID NO: 51 bestehenden Gruppe ausgewählte Oligonukleotid-Primer zur Beurteilung von rs962848 eingesetzt werden,
(viii) aus der aus SEQ ID NO: 60, SEQ ID NO: 61 und SEQ ID NO: 74 bestehenden Gruppe ausgewählte Oligonukleotid-Primer zur Beurteilung von rs4755233 eingesetzt werden,
(ix) aus der aus SEQ ID NO: 54, SEQ ID NO: 55 und SEQ ID NO: 71 bestehenden Gruppe ausgewählte Oligonukleotid-Primer zur Beurteilung von rs11037909 eingesetzt werden und
(x) aus der aus SEQ ID NO: 52, SEQ ID NO: 53 und SEQ ID NO: 70 bestehenden Gruppe ausgewählte Oligonukleotid-Primer zur Beurteilung von rs3740878 eingesetzt werden.

## Revendications

1. Procédé pour diagnostiquer une prédisposition au diabète de type 2 chez un sujet humain, comprenant les étapes consistant à :
a) fournir un échantillon biologique prélevé sur le sujet,
b) évaluer un ou plusieurs marqueur(s) SNP dans ledit échantillon, dans lequel lesdits marqueurs SNP sont associés au gène EXT2 et sont situés entre les positions 43 678 152 et 44 345 353 sur le chromosome 11p11,
c) comparer les données des marqueurs SNP obtenues à l'étape b) avec les données des marqueurs SNP provenant de gens sains et malade, dans le but de diagnostiquer une prédisposition au diabète de type 2.

2. Procédé selon la revendication 1, dans lequel les marqueurs SNP associés au gène EXT2 sont choisis dans le groupe constitué par les rs546614, rs886196, rs2863032, rs1073368, rs3814767, rs4379834, rs962848, rs4755233, rs11037909 et rs3740878.

3. Procédé, selon la revendication 1 ou la 2, comprenant en outre l'étape d) consistant à combiner des informations personnelles et cliniques avec les données des marqueurs SNP pour effectuer un diagnostic d'une prédisposition au diabète de type 2.

4. Procédé selon la revendication 3, dans lequel les informations personnelles et cliniques concernent l'âge, le genre, l'obésité, les antécédents familiaux d'obésité et de diabète, le rapport entre les tours de taille et de hanche (cm/cm) ainsi que les antécédents médicaux concernant le diabète du sujet.

5. Procédé, selon les revendications 3 ou 4, comprenant en outre la détermination des concentrations sanguines, sériques ou plasmatiques en fibrinogène, ferritine, récepteur de transferrine, protéine C réactive et insuline provenant du sujet.

6. Procédé selon l'une quelconque des revendications 1 à 5, dans lequel :
(i) des amorces d'oligonucléotides choisies dans le groupe constitué par les SEQ ID n° : 19, SEQ ID n° : 20 et SEQ ID n° : 44 sont utilisées pour évaluer le rs546614,
(ii) des amorces d'oligonucléotides choisies dans le groupe constitué par les SEQ ID n° : 21, SEQ ID n° : 22 et SEQ ID n° : 45 sont utilisées pour évaluer le rs886196,
(iii) des amorces d'oligonucléotides choisies dans le groupe constitué par les SEQ ID n° : 23, SEQ ID n° : 24 et SEQ ID n° : 46 sont utilisées pour évaluer le rs2863032,
(iv) des amorces d'oligonucléotides choisies dans le groupe constitué par les SEQ ID n° : 27, SEQ ID n° : 28 et SEQ ID n° : 48 sont utilisées pour évaluer le rs1073368,
(v) des amorces d'oligonucléotides choisies dans le groupe constitué par les SEQ ID n° : 29, SEQ ID n° : 30 et SEQ ID n° : 49 sont utilisées pour évaluer le rs3814767,
(vi) des amorces d'oligonucléotides choisies dans le groupe constitué par les SEQ ID n° : 31, SEQ ID n° : 32 et SEQ ID n° : 50 sont utilisées pour évaluer le rs4379834,
(vii) des amorces d'oligonucléotides choisies dans le groupe constitué par les SEQ ID n° : 33, SEQ ID n° : 34 et SEQ ID n° : 51 sont utilisées pour évaluer le rs962848,
(viii) des amorces d'oligonucléotides choisies dans le groupe constitué par les SEQ ID n° : 60, SEQ ID n° : 61 et SEQ ID n° : 74 sont utilisées pour évaluer le rs4755233,
(ix) des amorces d'oligonucléotides choisies dans le groupe constitué par les SEQ ID n° : 54, SEQ ID n° : 55 et SEQ ID n° : 71 sont utilisées pour évaluer le rs11037909 et
(x) des amorces d'oligonucléotides choisies dans le groupe constitué par les SEQ ID n° : 52, SEQ ID n° : 53 et SEQ ID n° : 70 sont utilisées pour évaluer le rs3740878.


### SEQUENCE LISTING

<110> Salonen, Jukka T.
   Fuentes, Ricardo
   Kontkanen, Outi
   Pirskanen, Mia
   Uimari, Pekka
   Aalto, Juha-Matti
<120> Method for detecting the risk of and for treatment of type 2 diabetes
<130>
<140>
   <141>
<150> US60/588,345
   <151> 2004-07-16
<160> 100
<170> PatentIn version 3.1
<210> 1
   <211> 22
   <212> DNA
   <213> Homo sapiens
<220>
   <221> misc_feature
   <223> PCR primer rs7106967 -F
<400> 1
   ggttacagag ttatgatagc ag 22
<210> 2
   <211> 20
   <212> DNA
   <213> Homo sapiens
<220>
   <221> misc_feature
   <223> PCR primer rs7106967 -R
<400> 2
   tttcacgaat tagtcttacc 20
<210> 3
   <211> 21
   <212> DNA
   <213> Homo sapiens
<220>
   <221> misc_feature
   <223> PCR promer rs4755736 -F
<400> 3
   gtcaagcttg cagagaatta c 21
<210> 4
   <211> 22
   <212> DNA
   <213> Homo sapiens
<220>
   <221> misc_feature
   <223> PCR primer rs4755736 -R
<400> 4
   agcagcacaa atgaactaag ac 22
<210> 5
   <211> 21
   <212> DNA
   <213> Homo sapiens
<220>
   <221> misc_feature
   <223> PCR primer rs4755741-F
<400> 5
   tggctatctc tgggcagtaa g 21
<210> 6
   <211> 21
   <212> DNA
   <213> Homo sapiens
<220>
   <221> misc_feature
   <223> PCR primer rs4755741-R
<400> 6
   ggggaatgat ttggacagta a 21
<210> 7
   <211> 21
   <212> DNA
   <213> Homo sapiens
<220>
   <221> misc_feature
   <223> PCR primer rs1878851 -F
<400> 7
   ctgatatttt tgatttgtct c 21
<210> 8
   <211> 20
   <212> DNA
   <213> Homo sapiens
<220>
   <221> misc_feature
   <223> PCR primer rs1878851 -R
<400> 8
   tcaatcttta tgtgcccttc 20
<210> 9
   <211> 20
   <212> DNA
   <213> Homo sapiens
<220>
   <221> misc_feature
   <223> PCR primer rs6485464 -F
<400> 9
   tgtcttcttt caggcaaatg 20
<210> 10
   <211> 21
   <212> DNA
   <213> Homo sapiens
<220>
   <221> misc_feature
   <223> PCR primer rs6485464 -R
<400> 10
   caacactatg gacagagaag g 21
<210> 11
   <211> 21
   <212> DNA
   <213> Homo sapiens
<220>
   <221> misc_feature
   <223> PCR primer rs1518820 -F
<400> 11
   atcatagcag tggaaagaga c 21
<210> 12
   <211> 20
   <212> DNA
   <213> Homo sapiens
<220>
   <221> misc_feature
   <223> PCR primer rs1518820 -R
<400> 12
   atggtttaaa atcaaggcag 20
<210> 13
   <211> 21
   <212> DNA
   <213> Homo sapiens
<220>
   <221> misc_feature
   <223> PCR primer rs1518818 -F
<400> 13
   agcagggctt ttggtgacag a 21
<210> 14
   <211> 21
   <212> DNA
   <213> Homo sapiens
<220>
   <221> misc_feature
   <223> PCR primer rs1518818 -R
<400> 14
   ctgtggcatg cagctgattt t 21
<210> 15
   <211> 20
   <212> DNA
   <213> Homo sapiens
<220>
   <221> misc_feature
   <223> PCR primer rs2292889 -F
<400> 15
   gaaacggagc aaaccttcca 20
<210> 16
   <211> 20
   <212> DNA
   <213> Homo sapiens
<220>
   <221> misc_feature
   <223> PCR primer rs2292889 -R
<400> 16
   tccccagagg cacaagtcca 20
<210> 17
   <211> 21
   <212> DNA
   <213> Homo sapiens
<220>
   <221> misc_feature
   <223> PCR primer rs2056248 -F
<400> 17
   ccccagggaa aagcagagga g 21
<210> 18
   <211> 21
   <212> DNA
   <213> Homo sapiens
<220>
   <221> misc_feature
   <223> PCR primer rs2056248 -R
<400> 18
   gggacccatt cacaggagta g 21
<210> 19
   <211> 20
   <212> DNA
   <213> Homo sapiens
<220>
   <221> misc_feature
   <223> PCR primer rs546614 -F
<400> 19
   cgctgccata atggaaacct 20
<210> 20
   <211> 20
   <212> DNA
   <213> Homo sapiens
<220>
   <221> misc_feature
   <223> PCR primer rs546614-R
<400> 20
   tgccttttcc tttcatctct 20
<210> 21
   <211> 21
   <212> DNA
   <213> Homo sapiens
<220>
   <221> misc_feature
   <223> PCR primer rs886196 -F
<400> 21
   tgtgggattt ctgtaggaga t 21
<210> 22
   <211> 22
   <212> DNA
   <213> Homo sapiens
<220>
   <221> misc_feature
   <223> PCR primer rs886196 -R
<400> 22
   gcagcaaaga acatgaatag gt 22
<210> 23
   <211> 20
   <212> DNA
   <213> Homo sapiens
<220>
   <221> misc_feature
   <223> PCR primer rs2863032 -F
<400> 23
   ctgggacatg caagaaaaag 20
<210> 24
   <211> 21
   <212> DNA
   <213> Homo sapiens
<220>
   <221> misc_feature
   <223> PCR primer rs2863032 -R
<400> 24
   cagaatttcc atgaacataa c 21
<210> 25
   <211> 21
   <212> DNA
   <213> Homo sapiens
<220>
   <221> misc_feature
   <223> PCR primer rs7942915 -F
<400> 25
   aagggtgtct cagatctgtg t 21
<210> 26
   <211> 21
   <212> DNA
   <213> Homo sapiens
<220>
   <221> misc_feature
   <223> PCR primer rs7942915 -R
<400> 26
   gatagggaga ccgagtaagt g 21
<210> 27
   <211> 21
   <212> DNA
   <213> Homo sapiens
<220>
   <221> misc_feature
   <223> PCR primer rs1073368-F
<400> 27
   cacccagccg actaattctt t 21
<210> 28
   <211> 21
   <212> DNA
   <213> Homo sapiens
<220>
   <221> misc_feature
   <223> PCR primer rs1073368-R
<400> 28
   aagacatgcc ccaatgaaca c 21
<210> 29
   <211> 22
   <212> DNA
   <213> Homo sapiens
<220>
   <221> misc_feature
   <223> PCR primer rs3814767 -F
<400> 29 22
   ggatacagtt ccagtggtga tt 22
<210> 30
   <211> 20
   <212> DNA
   <213> Homo sapiens
<220>
   <221> misc_feature
   <223> PCR primer rs3814767 -R
<400> 30
   ggggatggga cactcatgtt 20
<210> 31
   <211> 21
   <212> DNA
   <213> Homo sapiens
<220>
   <221> misc_feature
   <223> PCR primer rs4379834 -F
<400> 31
   tactggctgc ttcccttaaa c 21
<210> 32
   <211> 21
   <212> DNA
   <213> Homo sapiens
<220>
   <221> misc_feature
   <223> PCR primer rs4379834 -R
<400> 32
   gcttcccatc atcagatact t 21
<210> 33
   <211> 21
   <212> DNA
   <213> Homo sapiens
<220>
   <221> misc_feature
   <223> PCR primer rs962848 -F
<400> 33
   tgctttgcca tgtaggttat t 21
<210> 34
   <211> 21
   <212> DNA
   <213> Homo sapiens
<220>
   <221> misc_feature
   <223> PCR primer rs962848 -R
<400> 34
   aaggaggcta aagagacatg a 21
<210> 35
   <211> 24
   <212> DNA
   <213> Homo sapiens
<220>
   <221> misc_feature
   <223> Snapshot primer rs7106967 -R
<400> 35
   ttttttgtta gcctgttacc aata 24
<210> 36
   <211> 28
   <212> DNA
   <213> Homo sapiens
<220>
   <221> misc_feature
   <223> Snapshot primer rs4755736 -F
<400> 36
   tttttttttt cttgtctctg tttcagtc 28
<210> 37
   <211> 24
   <212> DNA
   <213> Homo sapiens
<220>
   <221> misc_feature
   <223> Snapshot primer rs4755741 -F
<400> 37
   tttttttgcc aacaatttta ggga 24
<210> 38
   <211> 32
   <212> DNA
   <213> Homo sapiens
<220>
   <221> misc_feature
   <223> Snapshot primer rs1878851 SS-F
<400> 38
   tttttttttt ttttagtatt tttgacccgt ca 32
<210> 39
   <211> 28
   <212> DNA
   <213> Homo sapiens
<220>
   <221> misc_feature
   <223> Snpshot primer rs6485464 -F
<400> 39
   tttttttttg ttaacaaagt gtgattac 28
<210> 40
   <211> 36
   <212> DNA
   <213> Homo sapiens
<220>
   <221> misc_feature
   <223> Snapshot primer rs1518820 -R
<400> 40
   tttttttttt ttttttttag ggaaattctt gcactt 36
<210> 41
   <211> 32
   <212> DNA
   <213> Homo sapiens
<220>
   <221> misc_feature
   <223> Snapshot primer rs1518818 -R
<400> 41
   tttttttttt ttttaaggtg gttgtgttaa ta 32
<210> 42
   <211> 40
   <212> DNA
   <213> Homo sapiens
<220>
   <221> misc_feature
   <223> Snapshot primer rs2292889 -F
<400> 42
   tttttttttt tttttttttt ttctaaccac agctcaaaat 40
<210> 43
   <211> 44
   <212> DNA
   <213> Homo sapiens
<220>
   <221> misc_feature
   <223> Snapshot primer rs2056248 -F
<400> 43
   tttttttttt tttttttttt ttttttccac cttgatgtgc atcc 44
<210> 44
   <211> 36
   <212> DNA
   <213> Homo sapiens
<220>
   <221> misc_feature
   <223> Snapshot primer rs546614 -F
<400> 44
   tttttttttt tttttttttg caaagaaaag agatga 36
<210> 45
   <211> 40
   <212> DNA
   <213> Homo sapiens
<220>
   <221> misc_feature
   <223> SNpshot primer rs886196 -F
<400> 45
   tttttttttt tttttttttt tttatcacag gatcttatca 40
<210> 46
   <211> 48
   <212> DNA
   <213> Homo sapiens
<220>
   <221> misc_feature
   <223> Snapshot primer rs2863032 SS-R
<400> 46
   tttttttttt tttttttttt tttttttttt attatattct aggcttgg 48
<210> 47
   <211> 44
   <212> DNA
   <213> Homo sapiens
<220>
   <221> misc_feature
   <223> Snapshot primer rs7942915 -F
<400> 47
   tttttttttt tttttttttt ttttttcagt ccatgctacc ttca 44
<210> 48
   <211> 52
   <212> DNA
   <213> Homo sapiens
<220>
   <221> misc_feature
   <223> Snapshot primer rs1073368 -R
<400> 48
   tttttttttt tttttttttt tttttttttt ttttgctgag aaacttattg ta 52
<210> 49
   <211> 52
   <212> DNA
   <213> Homo sapiens
<220>
   <221> misc_feature
   <223> Snapshot primer rs3814767 SS-R
<400> 49
   tttttttttt tttttttttt tttttttttt ttttcaaaat gtagcacaca cc 52
<210> 50
   <211> 56
   <212> DNA
   <213> Homo sapiens
<220>
   <221> misc_feature
   <223> Snapshot primer rs4379834 -R
<400> 50
   tttttttttt tttttttttt tttttttttt tttttttttc ttcctgtgaa gtagac 56
<210> 51
   <211> 60
   <212> DNA
   <213> Homo sapiens
<220>
   <221> misc_feature
   <223> Snapshot primer rs962848 -R
<400> 51
   tttttttttt tttttttttt tttttttttt tttttttttt ttcttcaccc agattcttca 60
<210> 52
   <211> 20
   <212> DNA
   <213> Homo sapiens
<220>
   <221> misc_feature
   <223> PCR primer exon 12 -F
<400> 52
   tgaatggagg aatggcgagg 20
<210> 53
   <211> 21
   <212> DNA
   <213> Homo sapiens
<220>
   <221> misc_feature
   <223> PCR primer exon 12 -R
<400> 53
   gggtgacctg ggcttgaact a 21
<210> 54
   <211> 22
   <212> DNA
   <213> Homo sapiens
<220>
   <221> misc_feature
   <223> PCR primer exon 11 -F
<400> 54
   catgggattt acagtagtag ac 22
<210> 55
   <211> 20
   <212> DNA
   <213> Homo sapiens
<220>
   <221> misc_feature
   <223> PCR primer exon 11 -R
<400> 55
   cgcatcaatc atagaacctt 20
<210> 56
   <211> 21
   <212> DNA
   <213> Homo sapiens
<220>
   <221> misc_feature
   <223> PCR primer exon 10 -F
<400> 56
   caaatcaggg cagttgagtt g 21
<210> 57
   <211> 21
   <212> DNA
   <213> Homo sapiens
<220>
   <221> misc_feature
   <223> PCR primer exon 10 -R
<400> 57
   agcacctgaa tgataaaatg g 21
<210> 58
   <211> 22
   <212> DNA
   <213> Homo sapiens
<220>
   <221> misc_feature
   <223> PCR primer exon 9 -F
<400> 58
   atctcccctg acacagttct ac 22
<210> 59
   <211> 21
   <212> DNA
   <213> Homo sapiens
<220>
   <221> misc_feature
   <223> PCR primer exon 9 -R
<400> 59
   cgccagcttc ttcacttatt g 21
<210> 60
   <211> 21
   <212> DNA
   <213> Homo sapiens
<220>
   <221> misc_feature
   <223> PCR primer exon 8 -F
<400> 60
   gttctcagct ccttttccag t 21
<210> 61
   <211> 22
   <212> DNA
   <213> Homo sapiens
<220>
   <221> misc_feature
   <223> PCR primer exon 8 -R
<400> 61
   caccctagaa caagaatgag at 22
<210> 62
   <211> 21
   <212> DNA
   <213> Homo sapiens
<220>
   <221> misc_feature
   <223> PCR primer exon 7 -F
<400> 62
   ggcaccccca tccctacaac t 21
<210> 63
   <211> 21
   <212> DNA
   <213> Homo sapiens
<220>
   <221> misc_feature
   <223> PCR primer exon 7 -R
<400> 63
   gcctctgcca caatcttgag c 21
<210> 64
   <211> 22
   <212> DNA
   <213> Homo sapiens
<220>
   <221> misc_feature
   <223> PCR primer exon 5 -F
<400> 64
   tagtacacta gggcctaaag ag 22
<210> 65
   <211> 22
   <212> DNA
   <213> Homo sapiens
<220>
   <221> misc_feature
   <223> PCR primer exon 5 -R
<400> 65
   ctgctctaga ccagtgtact aa 22
<210> 66
   <211> 21
   <212> DNA
   <213> Homo sapiens
<220>
   <221> misc_feature
   <223> PCR primer exon 4 -F
<400> 66
   cagtggaggt gaagactggt a 21
<210> 67
   <211> 21
   <212> DNA
   <213> Homo sapiens
<220>
   <221> misc_feature
   <223> PCR primer exon 4 -R
<400> 67
   catgtccagt aaagagcaat g 21
<210> 68
   <211> 21
   <212> DNA
   <213> Homo sapiens
<220>
   <221> misc_feature
   <223> PCR primer exon 3 -F
<400> 68
   ccaaccagtc ttcccatgca g 21
<210> 69
   <211> 21
   <212> DNA
   <213> Homo sapiens
<220>
   <221> misc_feature
   <223> PCR primer exon 3 -R
<400> 69
   agggaaacca cataggaagc c 21
<210> 70
   <211> 18
   <212> DNA
   <213> Homo sapiens
<220>
   <221> misc_feature
   <223> exon 12 RS
<400> 70
   tgctgtcctt atatcttc 18
<210> 71
   <211> 18
   <212> DNA
   <213> Homo sapiens
<220>
   <221> misc_feature
   <223> exon 11 RS
<400> 71
   cgcatcaatc atagaacc 18
<210> 72
   <211> 18
   <212> DNA
   <213> Homo sapiens
<220>
   <221> misc_feature
   <223> exon 10 FS
<400> 72
   atcccattat gaccttct 18
<210> 73
   <211> 18
   <212> DNA
   <213> Homo sapiens
<220>
   <221> misc_feature
   <223> exon 9 FS
<400> 73
   gatacaagct gattctcc 18
<210> 74
   <211> 18
   <212> DNA
   <213> Homo sapiens
<220>
   <221> misc_feature
   <223> exon 8 FS
<400> 74
   gttctcagct ccttttcc 18
<210> 75
   <211> 18
   <212> DNA
   <213> Homo sapiens
<220>
   <221> misc_feature
   <223> exon 7 FS
<400> 75
   gaattagcct aacctgga 18
<210> 76
   <211> 19
   <212> DNA
   <213> Homo sapiens
<220>
   <221> misc_feature
   <223> exon 5 FS
<400> 76 19
   aacccttgta gaaactttg 19
<210> 77
   <211> 19
   <212> DNA
   <213> Homo sapiens
<220>
   <221> misc_feature
   <223> exon 4 FS
<400> 77 19
   aggtgaagac tggtaagga 19
<210> 78
   <211> 19
   <212> DNA
   <213> Homo sapiens
<220>
   <221> misc_feature
   <223> exon 3 RS
<400> 78
   ccctgtaact gatgtattg 19
<210> 79
   <211> 51
   <212> DNA
   <213> Homo sapiens
<220>
   <221> misc_feature
   <223> rs7106967; R is A or G
<400> 79
   gagatcaaaa agatgtcctt gaatartatt ggtaacaggc taacctttct t 51
<210> 80
   <211> 51
   <212> DNA
   <213> Homo sapiens
<220>
   <221> misc_feature
   <223> rs4755736; K is G or T
<400> 80
   ctggcttctt gtctctgttt cagtckggtg tgacaagagt gacccaactg g 51
<210> 81
   <211> 51
   <212> DNA
   <213> Homo sapiens
<220>
   <221> misc_feature
   <223> rs4755741; R is A or G
<400> 81
   aatattttgc caacaatttt agggarttta ggaagtttcc ttactgtcca a 51
<210> 82
   <211> 51
   <212> DNA
   <213> Homo sapiens
<220>
   <221> misc_feature
   <223> rs1878851; Y is C or T
<400> 82
   tctccccagt atttttgacc cgtcaytggt tgaatccatg aatgtggagc t 51
<210> 83
   <211> 51
   <212> DNA
   <213> Homo sapiens
<220>
   <221> misc_feature
   <223> rs6485464; Y is C or T
<400> 83
   caattggtta acaaagtgtg attacygtaa tgcttacttg attccttagg a 51
<210> 84
   <211> 51
   <212> DNA
   <213> Homo sapiens
<220>
   <221> misc_feature
   <223> rs1518820; K is G or T
<400> 84
   acagcgtttg agctaagtct taaatkaagt gcaagaattt ccctaattgt a 51
<210> 85
   <211> 51
   <212> DNA
   <213> Homo sapiens
<220>
   <221> misc_feature
   <223> rs1518818;K is G or T
<400> 85
   aggcaatctc atgcgttatc tcattktatt aacacaacca ccttataaga a 51
<210> 86
   <211> 51
   <212> DNA
   <213> Homo sapiens
<220>
   <221> misc_feature
   <223> rs2292889; S is C or G
<400> 86
   gttatcccta accacagctc aaaatsgcta tcatctttag gcaaattaaa a 51
<210> 87
   <211> 51
   <212> DNA
   <213> Homo sapiens
<220>
   <221> misc_feature
   <223> rs2056248; Y is C or T
<400> 87
   gttcttccca ccttgatgtg catccyggga cacactgcat ccctgctccc c 51
<210> 88
   <211> 51
   <212> DNA
   <213> Homo sapiens
<220>
   <221> misc_feature
   <223> rs546614; M is A or C
<400> 88
   gaaggtatgc aaagaaaaga gatgamgttc gcaaggactg gccagatttc a 51
<210> 89
   <211> 51
   <212> DNA
   <213> Homo sapiens
<220>
   <221> misc_feature
   <223> rs886196; R is A or G
<400> 89
   gcactcttat cacaggatct tatcartctt taaaaatcat tgccaattga g 51
<210> 90
   <211> 51
   <212> DNA
   <213> Homo sapiens
<220>
   <221> misc_feature
   <223> ra2863032; Y is C or T
<400> 90
   ttgtatggag atttgttttt aaacayccaa gcctagaata taattctgtt t 51
<210> 91
   <211> 51
   <212> DNA
   <213> Homo sapiens
<220>
   <221> misc_feature
   <223> rs7942915; Y is C or T
<400> 91
   acctgttcag tccatgctac cttcayttcc cacctggaca tctgctcaac a 51
<210> 92
   <211> 51
   <212> DNA
   <213> Homo sapiens
<220>
   <221> misc_feature
   <223> rs1073368; R is A or G
<400> 92
   gacagagatt tccttaaacg cctagrtaca ataagtttct cagcctttgc c 51
<210> 93
   <211> 51
   <212> DNA
   <213> Homo sapiens
<220>
   <221> misc_feature
   <223> rs3814767; R is A or G
<400> 93
   cacccatcca gttctcaaat ggggtrggtg tgtgctacat tttgtatcat t 51
<210> 94
   <211> 51
   <212> DNA
   <213> Homo sapiens
<220>
   <221> misc_feature
   <223> rs4379834; Y is C or T
<400> 94
   cctccctttg tattagaacc atggtygtct acttcacagg aagaactcca g 51
<210> 95
   <211> 51
   <212> DNA
   <213> Homo sapiens
<220>
   <221> misc_feature
   <223> EXT2 gene SNP IVS7+126T>C; Y is C or T
<400> 95
   ctttctaaga tgagagtgtg cttttyatac ttggggcctg ataagggcag c 51
<210> 96
   <211> 51
   <212> DNA
   <213> Homo sapiens
<220>
   <221> misc_feature
   <223> rs12791572; S is C or G
<400> 96
   gtctatttat tgcagagata agtaasgagg catgggtctt gttggaaatc a 51
<210> 97
   <211> 51
   <212> DNA
   <213> Homo sapiens
<220>
   <221> misc_feature
   <223> rs962848; Y is C or T
<400> 97
   agcaactgtg ataaaagata aacagytgaa gaatctgggt gaagagtaca c 51
<210> 98
   <211> 51
   <212> DNA
   <213> Homo sapiens
<220>
   <221> misc_feature
   <223> rs4755233; Y is C or T
<400> 98
   ctgggatctg tcctggtaaa agccaycaag cctgccatgt ttgggtttgc t 51
<210> 99
   <211> 51
   <212> DNA
   <213> Homo sapiens
<220>
   <221> misc_feature
   <223> rs11037909; Y is C or T
<400> 99
   gctatgctgc cccttattta tcagcyaaag ggaactgcta tttttgaata t 51
<210> 100
   <211> 51
   <212> DNA
   <213> Homo sapiens
<220>
   <221> misc_feature
   <223> rs3740878; R is A or G
<400> 100
   aaaaacatac ctggctgtca gtgtcragga caataaaatc atgcttgtga c 51